# EUROPEAN PATENT APPLICATION

(11) **EP 1 736 171 A1**
(43) Date of publication of application: **27.12.2006**
(21) Application number: 05727932.5
(22) Date of filing: 30.03.2005
(51) Int. Cl.: A61K 38/22, A23L 1/305, A61K 48/00, A61P 3/04, A61P 3/06, A61P 3/10, C07K 14/475, C12N 15/09

(54) **ANTIOBESITY DRUG**

(30) Priority: 05.04.2004 JP 2004111501; 03.12.2004 JP 2004351813
(71) Applicant: Astellas Pharma Inc., Tokyo 103-8411 (JP)
(72) Inventor: YASUNAGA, Kunio, Astellas Pharma Inc., Tokyo 103-8411 (JP); YAMAJI, Noboru, Astellas Pharma Inc., Tokyo 103-8411 (JP); SUDA, Toshio, Keio University School of Medicine, Tokyo 1608582 (JP); OIKE, Yuichi, Keio University School of Medicine, Tokyo 1608582 (JP)
(74) Representative: Bates, Philip Ian
(86) International application number: PCT/JP2005/006024
(87) International publication number: WO 2005/097171

(57) **Abstract**

An antiobesity agent, an antidiabetic agent, and/or a hypolipidemic agent comprising an angiopoietin-related growth factor (AGF) or a polynucleotide encoding the same as an active ingredient a polypeptide are disclosed. Further, a functional food or a health food for alleviating obesity, diabetes, and/or hyperlipemia, comprising the AGF as an active ingredient is disclosed. Furthermore, a method for treating or preventing obesity, diabetes, and/or hyperlipemia, comprising administering to a subject the AGF or a polynucleotide encoding the same is disclosed. Furthermore, use of the AGF or a polynucleotide encoding the same in the manufacture of a medicament for treating obesity, diabetes, and/or hyperlipemia is disclosed.

## Description

### TECHNICAL FIELD

The present invention relates to an antiobesity agent comprising as an active ingredient an angiopoietin-related growth factor (hereinafter referred to as AGF) or a derivative thereof or a polynucleotide encoding the same. The antiobesity agent of the present invention may be administered as a medicament or in various forms, for example, eatable or drinkable products such as health foods (preferably functional foods) or feeds.

### BACKGROUND ART

Although the deleterious effect of obesity is widely known, there has been a remarkable increase in obesity in recent years. It is well-known that obesity (i.e., overaccumulation of fat in fatty tissues) causes various diseases, and thus it is proposed that obesity should be addressed as a disease to be treated. Diseases caused by obesity as a factor include, for example, lumbago, knee joint pain, and osteoarthrosis. Such orthopedic diseases are directly caused by a gain in body weight due to obesity. The overaccumulation of fat associated with obesity causes diabetes, hyperlipemia, hypertension, or arteriosclerotic disease. In particular, it is known that an overaccumulation of visceral fat is involved in the development of such diseases (non-patent reference 1).

Basic methods for alleviating obesity include kinesitherapy and diet therapy, but to continue with such therapy is difficult. As methods other than the kinesitherapy and diet therapy, medicaments are used. At present, Sibutramine and orlistat are mainly used on a global scale. However, these medicaments have not only a weak, but also an adverse effect. In Japan, only mazindol is authorized, but the application thereof is limited to severe obesity, and the period of administration is also limited (non-patent reference 2).

Due to the modernization of society, the number of patients suffering from diabetes is rapidly increasing, not only in Japan but also globally. In particular, it is known that the development of type II diabetes having a number of patients is involved in obesity or overaccumulation of fat. As with obesity, treatments for type II diabetes include kinesitherapy and diet therapy, but medicaments are used because it is difficult to continue this therapy. Patients suffering from severe diabetes are treated with insulin, but the treatment with insulin has a risk of an adverse effect such as hypoglycemia. As oral hypoglycemic drugs, thiazolidinediones or sulfonylurea agents are mainly used. However, the thiazolidinediones have an adverse effect such as hepatopathy, edema, or heart failure, and the SU agents have an adverse effect such as the promotion of obesity, and thus, an agent for alleviating insulin resistance without an increase in body weight or such adverse effects is greatly desired (non-patent reference 3).

In the visceral fat of an obese patient suffering from diabetes, hypertrophied adipocytes are observed. Adipocytokines capable of promoting insulin resistance are produced and secreted from hypertrophied adipocytes, and act on adipocytes, myocytes, and/or hepatocyte close to the hypertrophied adipocytes to promote insulin resistance. In patients suffering from diabetes, adipose tissues change to tissues which are involved in the promotion of insulin resistance (non-patent references 4 and 5).

Leptin is well-known as a factor involved in the accumulation of adipose tissues which cause obesity or diabetes. Leptin is an inhibitory hormone for bodyweight gain, and it is known that a deficiency of leptin causes obesity by promoting the appetite and reducing energy consumption. The findings of such factors involved in the accumulation of adipose tissues and hypertrophy of adipocytes are very useful in developing therapeutic agents for diseases such as obesity, diabetes, or hyperlipemia (non-patent reference 6).

An angiopoietin-related growth factor (AGF) is a secretory protein having a coiled-coil domain at the N-terminal side and a fibrinogen-like domain at the C-terminal side. The AGF is identical with NL8 reported in patent reference 1. It is reported that when CHO cells stably expressing NL8 are subcutaneously implanted into a nude mouse, the CHO cells exhibit tumorigenicity. Transgenic mice in which AGF was overexpressed in epidermal cells utilizing a K14 promoter were used to find that AGF exhibits an angiogenetic activity, an epidermal cell proliferating activity, a chondrocyte proliferating activity, an activity of promoting wound healing, and a tissue generative activity (patent reference 1 and non-patent reference 7). However, other physiological functions of AGF were unknown.

[non-patent reference 1] Metabolism, (U.S.A.), 1987, vol. 36, p.54-59
[non-patent reference 2] Nippon Rinsho, 2003, vol. 61, supplement 6, "Obesity", p.649-654
[non-patent reference 3] Nippon Rinsho, 2002, vol. 60, supplement 9, Shin-jidai no Tounyoubyougaku 3, p.310-331
[non-patent reference 4] Igaku no ayumi, 2000, vol. 192, p.513-518
[non-patent reference 5] Igaku no ayumi, 2000, vol. 192, p.541-545
[non-patent reference 6] Trends in Molecular Medicine, (Netherlands), 2002, vol. 8, no. 9, p.442-447
[non-patent reference 7] Proceedings of the National Academy of Sciences of the United States of America, (U.S.A.), 2003, Vol. 100, p. 9494-9499
[patent reference 1] International Publication No. WO99/15653
[patent reference 2] International Publication No. WO03/083114

### DISCLOSURE OF INVENTION

### PROBLEMS TO BE SOLVED BY THE INVENTION

An object of the present invention is to provide a novel antiobesity agent.

### MEANS FOR SOLVING THE PROBLEMS

The present inventors have conducted intensive studies and, as a result, can now reveal that AGF has an antiobesity activity, an antidiabetic activity, and a hypolipidemic activity, by preparing and analyzing AGF knockout (KO) mice and AGF transgenic (Tg) mice. That is, the present inventors found that AGF knockout mice became remarkably obese (Example 3). Further, the present inventors found that, in the AGF knockout mice, there was an increase in weight of adipose tissues accompanied by obesity (Example 4), adipocytes were enlarged (Example 5), and there was an increase in each amount of triglyceride contained in skeletal muscles or liver (Example 6). In this connection, it is known that the triglyceride contents in skeletal muscles or liver are increased by obesity. In contrast, the present inventors found that, in the AGF transgenic mice (CAG-AGF Tg mice, in which AGF was systemically overexpressed), an increase in body weight was suppressed (Example 3), an increase in weight of adipose tissues was suppressed (Example 4), an enlargement of adipocytes was suppressed (Example 5), each amount of triglyceride contained in skeletal muscles or liver was decreased (Example 6), and insulin sensitivity, lipid metabolism, and glucose tolerance were improved (Examples 16 and 17). Further, it was shown that the AGF KO mice suffered from hyperinsulinemia and exhibited an abnormality in glucose tolerance (Referential Example 2). Furthermore, it was found that, in mice to which an adenovirus expressing AGF was administered, an increase in body weight was suppressed and glucose tolerance was improved (Examples 15 and 20), and that, in other AGF transgenic mice (K14-AGF Tg mice, in which AGF was overexpressed in epidermal cells), AGF exhibited an antiobesity activity, an activity of decreasing fat tissue, and an activity of increasing insulin sensitivity (Examples 18 and 19).
From these findings, the present inventors found that AGF can be used as an active ingredient of therapeutic agents for obesity, diabetes, and/or hyperlipemia, and thus the present invention was completed.

The present invention relates to
[1] an antiobesity agent, an antidiabetic agent, and/or a hypolipidemic agent comprising as an active ingredient a polypeptide selected from the group consisting of the following (a) to (d) or a polynucleotide encoding the polypeptide:
   (a) a polypeptide exhibiting an activity of suppressing an increase in body weight, and comprising the amino acid sequence consisting of amino acids 1-450 of SEQ ID NO: 3 or the amino acid sequence consisting of amino acids 1-433 of SEQ ID NO: 5,
   (b) a polypeptide exhibiting an activity of suppressing an increase in body weight, and comprising an amino acid sequence in which 1 to 10 amino acids are substituted, deleted and/or inserted in the amino acid sequence consisting of amino acids 1-450 of SEQ ID NO: 3 or the amino acid sequence consisting of amino acids 1-433 of SEQ ID NO: 5,
   (c) a polypeptide exhibiting an activity of suppressing an increase in body weight, and encoded by a DNA which hybridizes under stringent conditions to a DNA encoding the amino acid sequence consisting of amino acids 1-450 of SEQ ID NO: 3 or amino acids 1-433 of SEQ ID NO: 5, and
   (d) a polypeptide exhibiting an activity of suppressing an increase in body weight, and comprising an amino acid sequence having a 95% or more identity with that consisting of amino acids 1-450 of SEQ ID NO: 3 or amino acids 1-433 of SEQ ID NO: 5,
[2] a functional food or a health food for alleviating obesity, diabetes, and/or hyperlipemia, comprising the polypeptide of [1] as an active ingredient,
[3] a method for treating or preventing obesity, diabetes, and/or hyperlipemia, comprising administering to a subject in need thereof the polypeptide of [1] or a polynucleotide encoding the polypeptide in an amount effective therefor, and
[4] use of the polypeptide of [1] or a polynucleotide encoding the polypeptide in the manufacture of a medicament for treating obesity, diabetes, and/or hyperlipemia.

The term "transgenic animal" as used herein means an animal in which a promoter and a gene are introduced into a chromosome to overexpress the gene at a desired location. The term "knockout animal" as used herein means an animal in which an expression of a particular gene is deleted by gene manipulation of a chromosome.

### EFFECTS OF THE INVENTION

AGF is useful as an active ingredient of an antiobesity agent, an antidiabetic agent, and/or a hypolipidemic agent.

### BRIEF DESCRIPTION OF THE DRAWINGS

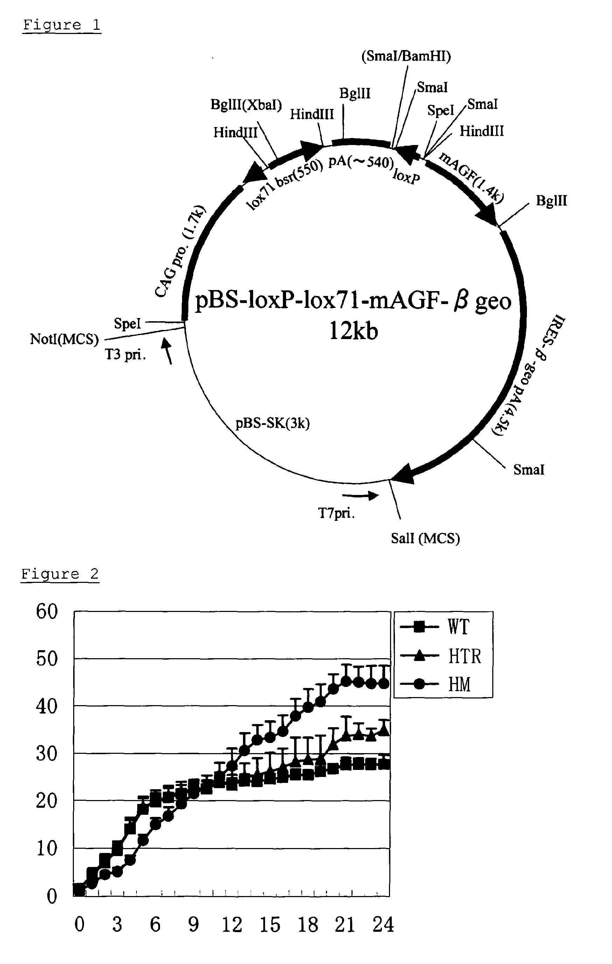

Figure 1 is a schematic drawing showing the structure of plasmid pBS-loxP-lox71-mAGF-pgeo. The abbreviations "pro." and "pri." mean a promoter and a primer, respectively.
Figure 2 is a graph showing changes in body weight of AGF KO mice. The horizontal axis indicates an age in weeks (weeks), and the vertical axis indicates body weight (g). The abbreviations "WT", "HTR", and "HM" mean WT mice, heterozygous KO mice, and homozygous KO mice, respectively.

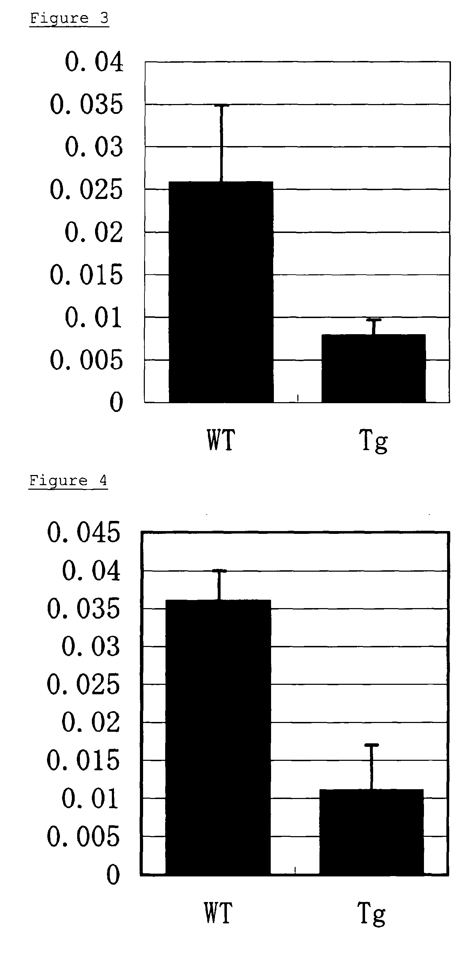

Figure 3 is a graph showing changes in the weight of genital fat pads (white adipose tissue) of CAG-AGF Tg mice (normal diet). The vertical axis indicates [weight of white adipose tissue (g)]/[body weight (g)]. The abbreviations "WT" and "Tg" mean WT mice and Tg mice, respectively.
Figure 4 is a graph showing changes in the weight of genital fat pads (white adipose tissue) of CAG-AGF Tg mice (high fat diet). The vertical axis indicates [weight of white adipose tissue (g)]/[body weight (g)]. The abbreviations "WT" and "Tg" mean WT mice and Tg mice, respectively.

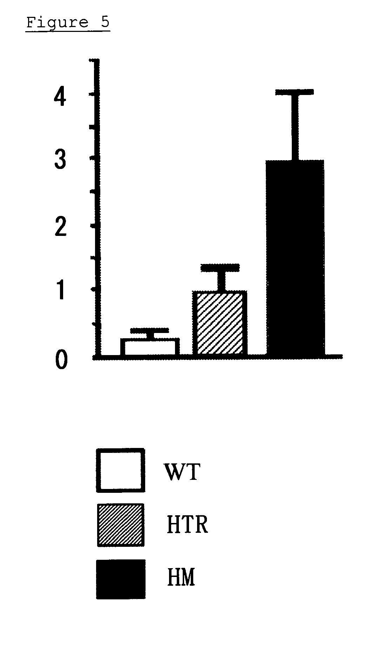

Figure 5 is a graph showing changes in the weight of genital fat pads (white adipose tissue) of AGF KO mice (weight of white adipose tissue). The vertical axis indicates the weight of white adipose tissue (g). The abbreviations "WT", "HTR", and "HM" mean WT mice, heterozygous KO mice, and homozygous KO mice, respectively.

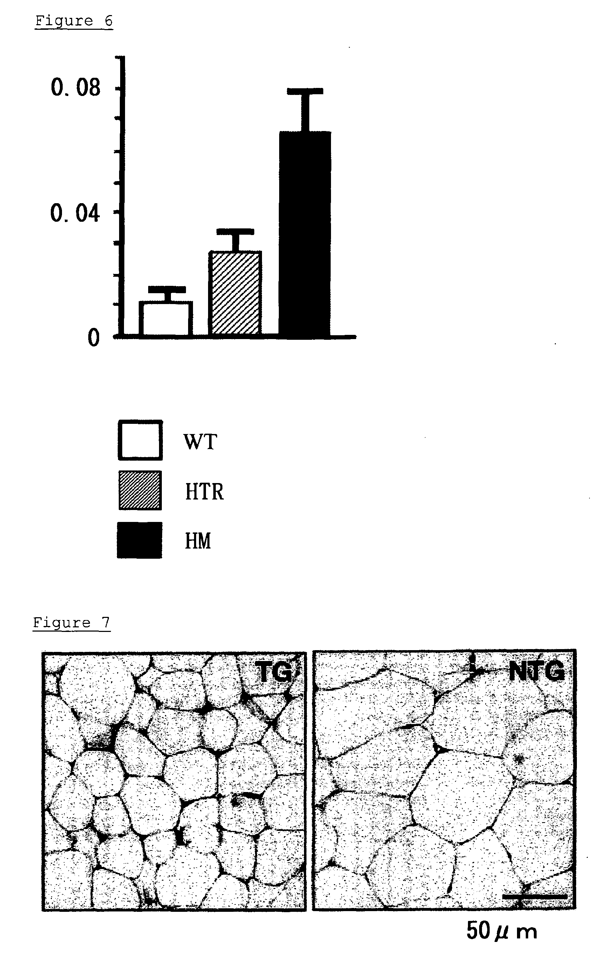

Figure 6 is a graph showing changes in the weight of genital fat pads (white adipose tissue) of AGF KO mice (weight of white adipose tissue/body weight). The vertical axis indicates [weight of white adipose tissue (g)]/[body weight (g)]. The abbreviations "WT", "HTR", and "HM" mean WT mice, heterozygous KO mice, and homozygous KO mice, respectively.
Figure 7 is a microphotograph showing the form of adipocytes in the CAG-AGF Tg mouse. The abbreviations "Tg" and "NTG" mean Tg mice and WT mice, respectively.

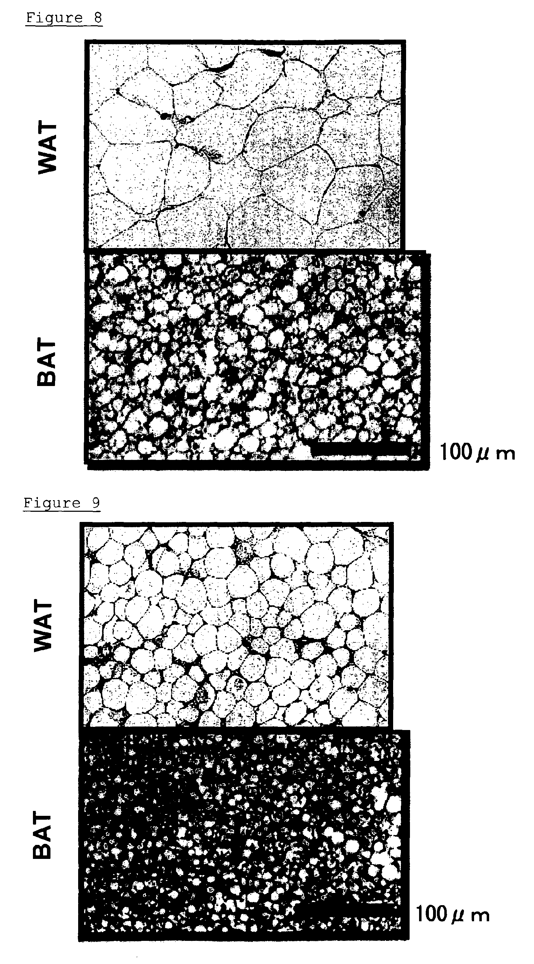

Figure 8 is a microphotograph showing the form of adipocytes in the AGF homozygous KO mouse.
Figure 9 is a microphotograph showing the form of adipocytes in the littermate WT mouse.

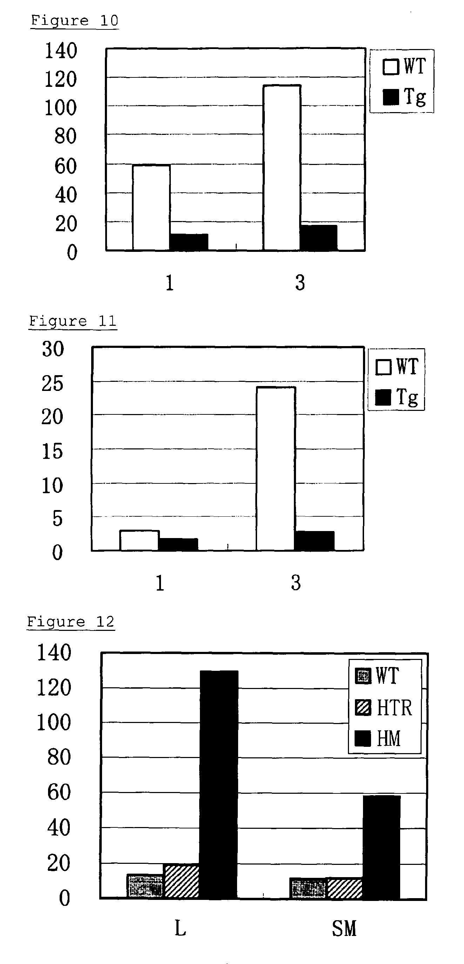

Figure 10 is a graph showing TG content in tissue (liver) of the CAG-AGF Tg mouse. The horizontal axis indicates the period of breeding with a high fat diet (month). The vertical axis indicates a TG content (mg/g tissue). The abbreviations "WT" and "Tg" mean WT mice and Tg mice, respectively.
Figure 11 is a graph showing TG content in tissue (skeletal muscles) of the CAG-AGF Tg mouse. The horizontal axis indicates the period of breeding with a high fat diet (months). The vertical axis indicates a TG content (mg/g tissue). The abbreviations "WT" and "Tg" mean WT mice and Tg mice, respectively.
Figure 12 is a graph showing TG content in tissue of the AGF KO mice. The vertical axis indicates a TG content (mg/g tissue). The abbreviations "L" and "SM" in the horizontal axis mean the liver and skeletal muscles, respectively. The abbreviations "WT", "HTR", and "HM" mean WT mice, heterozygous KO mice, and homozygous KO mice, respectively.

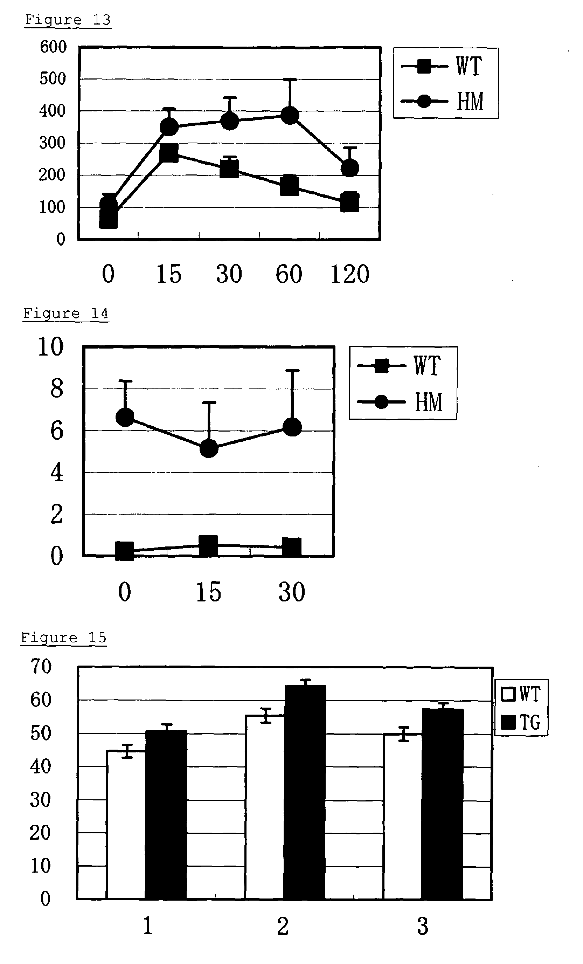

Figure 13 is a graph showing the result (blood glucose level) of a glucose tolerance test in the AGF KO mice. The horizontal axis indicates a time (minutes), and the vertical axis indicates a blood glucose level (mg/dL). The abbreviations "WT" and "HM" mean WT mice and homozygous KO mice, respectively.
Figure 14 is a graph showing the result (serum insulin) of a glucose tolerance test in the AGF KO mice. The horizontal axis indicates a time (minutes), and the vertical axis indicates serum insulin (ng/mL). The abbreviations "WT" and "HM" mean WT mice and homozygous KO mice, respectively.
Figure 15 is a graph showing oxygen consumption in the CAG-AGF Tg mice. In the horizontal axis, lane 1, lane 2, and lane 3 indicate results in the light period, the dark period, and 24 hours, respectively. The vertical axis indicates VO₂ (mL/kg/min). The abbreviations "WT" and "Tg" mean WT mice and Tg mice, respectively.

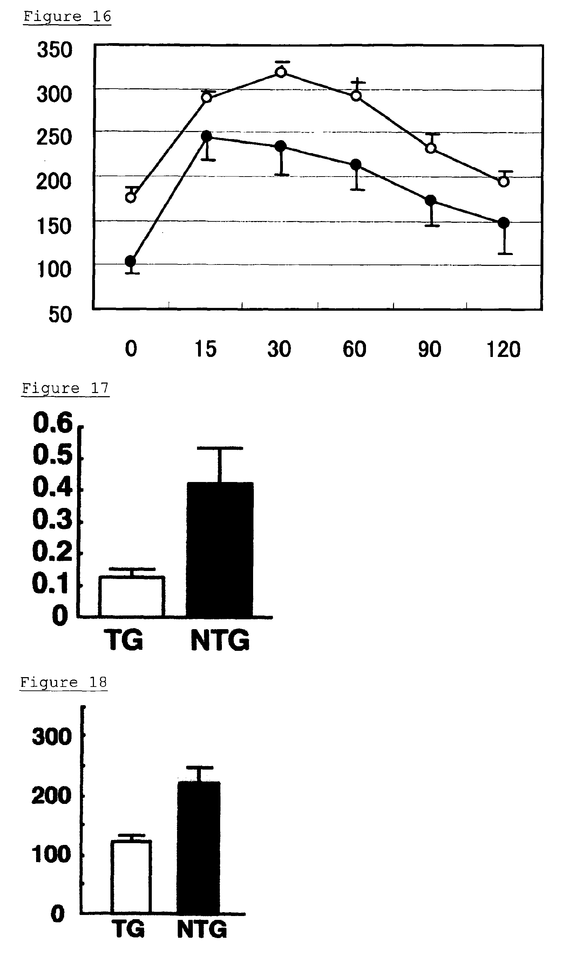

Figure 16 is a graph showing the result of a glucose metabolism test in mice to which an adenovirus expressing the mouse AGF was administered. The solid circles indicate the result from mAGF-Adeno administered mice, and the open circles indicate the result from Cont-Adeno administered mice. The horizontal axis indicates a time (minutes), and the vertical axis indicates blood glucose level (mg/dL).
Figure 17 is a graph showing a concentration of insulin in plasma (vertical axis; ng/mL) of CAG-AGF Tg mice and littermate WT mice bred with a high fat diet. The abbreviations "TG" and "NTG" mean the CAG-AGF Tg mice and the littermate WT mice, respectively.
Figure 18 is a graph showing a concentration of cholesterol in serum (vertical axis; mg/dL) of CAG-AGF Tg mice and littermate WT mice bred with a high fat diet. The abbreviations "TG" and "NTG" mean the CAG-AGF Tg mice and the littermate WT mice, respectively.

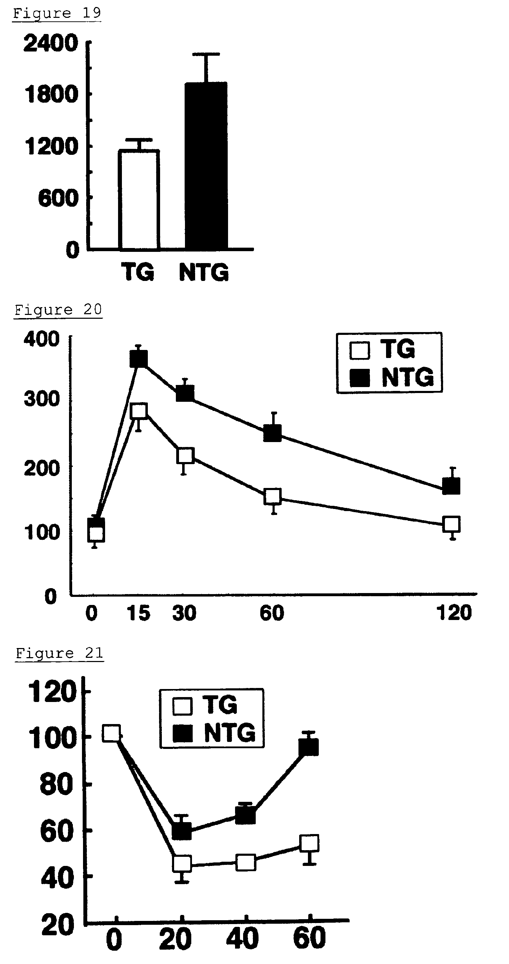

Figure 19 is a graph showing a concentration of free fatty acids in serum (vertical axis; µEq/L) of CAG-AGF Tg mice and littermate WT mice bred with a high fat diet. The abbreviations "TG" and "NTG" mean the CAG-AGF Tg mice and the littermate WT mice, respectively.
Figure 20 is a graph showing the result of a glucose metabolism test in CAG-AGF Tg mice and littermate WT mice. The abbreviations "TG" and "NTG" mean the CAG-AGF Tg mice and the littermate WT mice, respectively. The horizontal axis indicates a time (minutes), and the vertical axis indicates blood glucose level (mg/dL).
   Figure 21 is a graph showing the result of an insulin sensitivity test in CAG-AGF Tg mice and littermate WT mice. The abbreviations "TG" and "NTG" mean the CAG-AGF Tg mice and the littermate WT mice, respectively. The horizontal axis indicates a time (minutes), and the vertical axis indicates the ratio (%) of the blood glucose level after the insulin administration to that before the insulin administration.

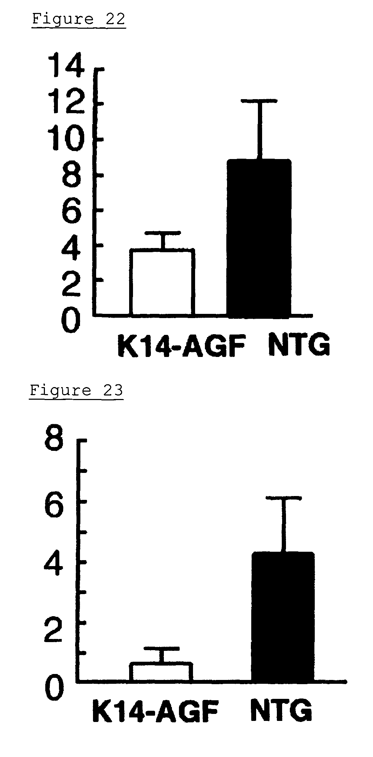

Figure 22 is a graph showing the weight of visceral fat tissue in K14-AGF Tg mice and littermate WT mice. The abbreviations "K14-AGF" and "NTG" mean the K14-AGF Tg mice and the littermate WT mice, respectively. The vertical axis indicates the percentage (%) of the weight of visceral fat tissue per body weight.
Figure 23 is a graph showing the weight of subcutaneous fat tissue in K14-AGF Tg mice and littermate WT mice. The abbreviations "K14-AGF" and "NTG" mean the K14-AGF Tg mice and the littermate WT mice, respectively. The vertical axis indicates the percentage (%) of the weight of subcutaneous fat tissue per body weight.

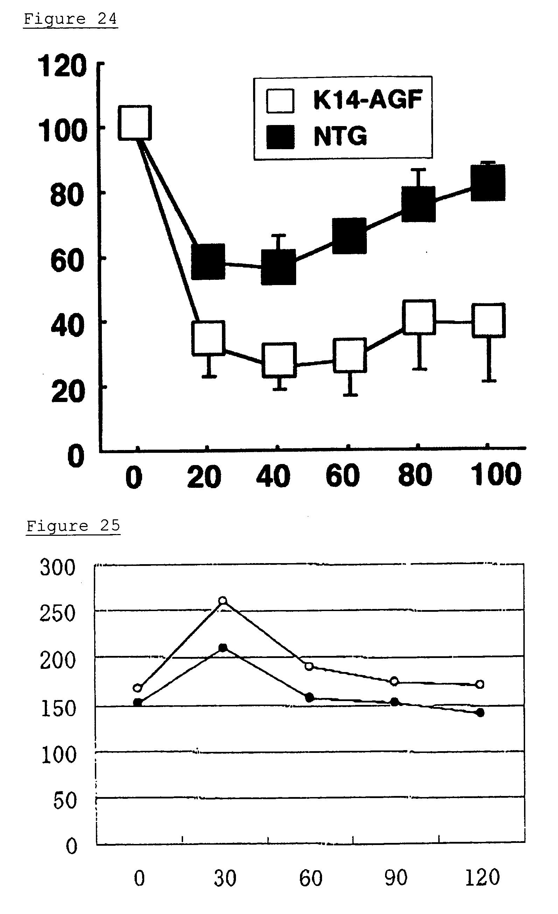

Figure 24 is a graph showing the result of an insulin sensitivity test in K14-AGF Tg mice and littermate WT mice. The abbreviations "K14-AGF" and "NTG" mean the K14-AGF Tg mice and the littermate WT mice, respectively. The horizontal axis indicates a time (minutes), and the vertical axis indicates the ratio (%) of the blood glucose level after the insulin administration to that before the insulin administration.
Figure 25 is a graph showing the result of a glucose metabolism test in CAG-AGF Tg mice and littermate WT mice. The solid circles indicate the result from hAGF-Adeno administered mice, and the open circles indicate the result from Cont-Adeno administered mice. The horizontal axis indicates a time (minutes), and the vertical axis indicates blood glucose level (mg/dL).

### BEST MODE FOR CARRYING OUT THE INVENTION

### [1] Medicament of the present invention

The antiobesity agent, the antidiabetic agent, and/or the hypolipidemic agent of the present invention (hereinafter referred to as the medicament of the present invention) may contain as an active ingredient at least a polypeptide selected from the group consisting of the following (a) to (d) or a polynucleotide encoding the polypeptide:
(a) a polypeptide exhibiting an activity of suppressing an increase in body weight, and comprising the amino acid sequence consisting of amino acids 1-450 of SEQ ID NO: 3 or the amino acid sequence consisting of amino acids 1-433 of SEQ ID NO: 5,
(b) a polypeptide exhibiting an activity of suppressing an increase in body weight, and comprising an amino acid sequence in which 1 to 10 amino acids are substituted, deleted and/or inserted in the amino acid sequence consisting of amino acids 1-450 of SEQ ID NO: 3 or the amino acid sequence consisting of amino acids 1-433 of SEQ ID NO: 5,
(c) a polypeptide exhibiting an activity of suppressing an increase in body weight, and encoded by a DNA which hybridizes under stringent conditions to a DNA encoding the amino acid sequence consisting of amino acids 1-450 of SEQ ID NO: 3 or amino acids 1-433 of SEQ ID NO: 5, and
(d) a polypeptide exhibiting an activity of suppressing an increase in body weight, and comprising an amino acid sequence having a 95% or more identity with that consisting of amino acids 1-450 of SEQ ID NO: 3 or amino acids 1-433 of SEQ ID NO: 5.

The term "antiobesity agent" as used herein includes both an agent used for treating a patient suffering from obesity and an agent used preventively for a subject exhibiting signs of obesity.
The term "antidiabetic agent" as used herein includes both an agent used for treating a patient suffering from diabetes and an agent used preventively for a subject exhibiting signs of diabetes.
The term "hypolipidemic agent" as used herein includes both an agent used for treating a patient suffering from hyperlipemia and an agent used preventively for a subject exhibiting signs of hyperlipemia.

As the polypeptide for the active ingredient of the medicament of the present invention, a human AGF consisting of the amino acid sequence consisting of amino acids 1-450 of SEQ ID NO: 3, or a mouse AGF consisting of the amino acid sequence consisting of amino acids 1-433 of SEQ ID NO: 5 is preferable.

The amino acid sequence of SEQ ID NO: 3 is that of a human AGF precursor. The human AGF has a signal sequence (-20 to -1) at the N terminus thereof, and the signal sequence is cleaved when the precursor is secreted to the outside of cells. A human matured AGF, which is generated by cleaving the signal sequence and is composed of the amino acid sequence consisting of amino acids 1-450 of SEQ ID NO: 3, has physiological activities.
Similarly, the amino acid sequence of SEQ ID NO: 5 is that of a mouse AGF precursor. The mouse AGF has a signal sequence (-24 to -1) at the N terminus thereof, and the signal sequence is cleaved when the precursor is secreted to the outside of cells. A mouse matured AGF, which is generated by cleaving the signal sequence and is composed of the amino acid sequence consisting of amino acids 1-433 of SEQ ID NO: 5, has physiological activities.

As the above polypeptide (b) which may be used as the active ingredient of the medicament of the present invention [i.e., a polypeptide exhibiting an activity of suppressing an increase in body weight, and comprising an amino acid sequence in which 1 to 10 (for example, one to several) amino acids are substituted, deleted, and/or inserted in an amino acid sequence consisting of amino acids 1-450 of SEQ ID NO: 3 or amino acids 1-433 of SEQ ID NO: 5], there may be mentioned, for example, a polypeptide exhibiting an activity of suppressing an increase in body weight, and comprising an amino acid sequence in which preferably 1 to 7, more preferably 1 to 5 amino acids are substituted, deleted, and/or inserted in an amino acid sequence consisting of amino acids 1-450 of SEQ ID NO: 3 or amino acids 1-433 of SEQ ID NO: 5.

To maintain the functions of the original polypeptide, the amino acid to be substituted is preferably an amino acid having properties similar to those of the original amino acid. For example, amino acids belonging to each of the following groups have properties similar to those of other members in the group. When these amino acids are substituted with other amino acids in the same group, the essential functions of the original protein are often maintained. Such amino acid substitution is called a conservative substitution, and is known as a method for changing an amino acid sequence while maintaining the polypeptide functions.
Nonpolar amino acids: Ala, Val, Leu, Ile, Pro, Met, Phe, and Trp
Uncharged amino acids: Gly, Ser, Thr, Cys, Tyr, Asn, and GIn
Acidic amino acids : Asp and Glu
Basic amino acids: Lys, Arg, and His

A method for judging whether or not a polypeptide of interest exhibits the activity of suppressing an increase in body weight is not limited, but it may be confirmed by, for example, the method described in Example 3. That is, the activity may be confirmed by breeding transgenic animals, prepared by utilizing a gene encoding the polypeptide, with a normal diet or a high fat diet, and comparing changes in body weight with those of wild-type animals. Alternatively, it may be confirmed by the method described in Example 15 or 20. That is, the activity may be confirmed by administering an adenovirus expressing the polypeptide to mice bred with a normal diet or a high fat diet, and comparing changes in body weight with those of mice to which a control adenovirus is administered.

With respect to the above polypeptide (c) which may be used as the active ingredient of the medicament of the present invention, the "stringent conditions" include, as hybridization conditions, conditions of "5 x SSPE, 5 x Denhard's solution, 0.5% sodium dodecyl sulfate (SDS), 40% formamide, and 200 µg/mL salmon sperm DNA, at 37°C overnight", and, as more stringent hybridization conditions, conditions of "5 x SSPE, 5 x Denhard's solution, 0.5% SDS, 50% formamide, and 200 µg/mL salmon sperm DNA, at 42°C overnight". Further, washing conditions include mild conditions such as "5 x SSC and 1% SDS, at 42°C", usual conditions such as "0.5 x SSC and 0.1% SDS, at 42°C", and more stringent conditions such as "0.2 x SSC and 0.1% SDS, at 65°C". The "5 x SSPE" contains 50 mmol/L sodium phosphate (pH 7.4), 0.75 mol/L NaCl, and 5 mmol/L EDTA. The "5 x SSC" contains 0.75 mol/L NaCl and 75 mmol/L sodium citrate (pH 7.0).

The homology in the above polypeptide (d) which may be used as the active ingredient of the medicament of the present invention is at least 95% or more, preferably 97% or more. The homology between amino acid sequences may be calculated by a BLAST search algorithm. More particularly, it may be calculated using a bl2seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap insertion cost value, "0" as a Gap elongation cost value, "SEG" as a filter for a Query sequence, and "BLOSUM62" as a Matrix are used, respectively.

As the polypeptide which may be used as the active ingredient of the medicament of the present invention, a polypeptide in which a signal sequence is added to the N terminus of any one of the polypeptides (a) to (d) is preferable. The signal sequence is not particularly limited, so long as it may lead a polypeptide to pass through the membrane. As the signal sequence, signal sequences described in Biochemistry, 28(3), 923-930, 1989 may be used. As the polypeptide which may be used as the active ingredient of the medicament of the present invention, a polypeptide in which a signal sequence (-20 to -1) in the amino acid sequence of SEQ ID NO: 3 or a signal sequence (-24 to -1) in the amino acid sequence of SEQ ID NO: 5 is added to the N terminus of any one of the polypeptides (a) to (d) is more preferable, and a polypeptide consisting of the amino acid sequence of SEQ ID NO: 3 or 5 is most preferable.

The origin of the polypeptide which may be used as the active ingredient of the medicament of the present invention is not limited to a human or a mouse. For example, a polypeptide derived from organisms other than a human or a mouse, or a polypeptide obtained, using genetic engineering techniques, by artificially modifying an amino acid sequence consisting of amino acids 1-450 of SEQ ID NO: 3 or amino acids 1-433 of SEQ ID NO: 5, may be used, so long as it is included in any one of the polypeptides (a) to (d). As the polypeptide, a recombinant polypeptide is preferable.

The polynucleotides encoding the polypeptides (a) to (d), i.e., the polynucleotides which may be used as the active ingredient of the medicament of the present invention, include DNAs and RNAs, and DNAs are preferable. As the polynucleotide, there may be mentioned, for example, a polynucleotide which encodes a polypeptide having an activity of suppressing an increase in body weight and contains a nucleotide sequence consisting of nucleotides 61-1410 of SEQ ID NO: 2 or nucleotides 73-1371 of SEQ ID NO: 4. A polynucleotide consisting of a nucleotide sequence of SEQ ID NO: 2 or 4, or a polynucleotide consisting of a nucleotide sequence consisting of nucleotides 61-1410 of SEQ ID NO: 2 or nucleotides 73-1371 of SEQ ID NO: 4, is preferable.

The present invention includes a method for treating or preventing obesity, diabetes, and/or hyperlipemia, comprising administering to a subject in need thereof at least one of the polypeptides (a) to (d) or polynucleotides encoding the polypeptides in an amount effective therefor. Further, the present invention includes a use of the polypeptides (a) to (d) or polynucleotides encoding the polypeptides in the manufacture of a medicament for treating obesity, diabetes, and/or hyperlipemia.

Examples of administration of the polypeptide as the medicament of the present invention include oral administration by tablets, pills, capsules, granules, fine granules, powders, oral solutions and the like, and parenteral administration by injections (e.g., intravenous, intramuscular, or the like), suppositories, transdermal preparations, transmucosal absorption preparations and the like. Particularly, in the case of polypeptides which are digested by enzymes, a parenteral administration such as transdermal preparation or intravenous injection or the like, or administration using formulations in which the polypeptide is delivered without digestion to a lower gastrointestinal tract (such as jejunum, ileum, colon, or large intestine) where digestive enzymes are not very effective, is desirable.

The medicament containing at least one of the polypeptides (a) to (d) as the active ingredient may be prepared as a pharmaceutical composition by using pharmaceutically acceptable carriers, fillers, or other additives which may be commonly used in polypeptide formulations.

The injections for parenteral administration may include aseptic aqueous or non-aqueous solutions, suspensions, and emulsions. Examples of the diluent for use in the aqueous solutions and suspensions include distilled water for injection use and physiological saline. Examples of the diluent for use in the non-aqueous solutions and suspensions include propylene glycol, polyethylene glycol, vegetable oils such as olive oil, alcohols such as ethanol, polysorbate 80, and the like. Such a composition may further contain a moistening agent, an emulsifying agent, a dispersing agent, a stabilizing agent, a solubilizing or solubilization assisting agent, an antiseptic or the like. These compositions may be sterilized, for example, by filtration through a bacteria retaining filter, blending of a germicide, or irradiation. Alternatively, they may be used by first making them into sterile solid compositions and dissolving them in sterile water or other sterile solvent for injection use prior to their use.

The medicament of the present invention contains at least one of the polypeptides (a) to (d) in an amount sufficient to cause desired effects for the treatment or prevention of obesity, diabetes, and/or hyperlipemia, i.e., in an amount clinically or pharmaceutically effective therefor. The effective amount in an actual administration may be confirmed, for example, by measuring a degree of recovery from a disease of interest. The actual dose depends on various factors, such as symptoms, age, body weight of each subject to be administered, and preferably may be determined by taking into consideration any noticeable side effects. The dose, toxicity or the like may be easily determined by those skilled in the art by using a known model animal. For example, the usual dosage for an adult is preferably 1 µg to 2 g per day, more preferably 1 µg to 200 mg per day.

Instead of the polypeptides (a) to (d), at least one of polynucleotides encoding the polypeptides may be administered in a gene therapy, as an antiobesity agent, an antidiabetic agent, and/or a hypolipidemic agent. General administrations in a gene therapy may be broadly divided into methods using a nonvirus vector and methods using a virus vector ["Bessatsu Jikken igaku, Idenshi chiryo no kiso gijyutsu", Yodo-sha, 1996; "Bessatsu Jikken igaku, Idenshi donyu & hatsugen kaiseki jikken-hou", Yodo-sha, 1997; and The Japan Society of Gene Therapy ed., "Idenshi chiryo kaihatsu kenkyu handbook", NTS, 1999].

With respect to the methods using a nonvirus vector, as methods for introducing a gene into a tissue, there may be mentioned, for example, a method using an encapsulating liposome, a method using an electrostatic liposome, a method using HVJ-liposome [for example, J.Clin.Invest. 93: 1458-1464 (1994)], a method utilizing receptor-mediated introduction, a method in which a particle gun is used to introduce the polynucleotide together with a carrier such as a metal particle to cells, a direct introduction method of a plasmid, or an introduction method using a cationic polymer. As methods for introducing a gene into cells, for example, lipofection, calcium phosphate coprecipitation, a DEAE-dextran method, or direct injection of DNA using a microcapillary are known.

As the virus vector, there may be mentioned, for example, a retrovirus vector, an adenovirus vector, or an adeno-associated virus vector. A polynucleotide encoding the polypeptides (a) to (d) may be introduced into cells by introducing the polynucleotide into a DNA or RNA virus, such as a detoxicated retrovirus, adenovirus, adeno-associated virus, herpes virus, vaccinia virus, poxvirus, poliovirus, Sindbis virus, SV40, HIV, or Sendai virus, and infecting the cells with such virus.

The therapeutic agent for obesity, diabetes, or hyperlipemia comprising a polynucleotide encoding the polypeptides (a) to (d) may be administered by an in vivo method in which a gene is directly introduced into a living body, or by an ex vivo method in which a gene is introduced into cells taken from a living body and the gene-introduced cells are implanted into the living body.

As the formulations for the in vivo method, liquid formulations for an injection or infusion may be used. Liquid formulations may be prepared, for example, by dissolving the polynucleotide in a solvent, such as buffers [for example, phosphate-buffered saline (PBS)], physiological saline, or distilled water, sterilizing the solution by filtration or the like if desired, and filling a sterilized container therewith. If necessary, the solution can be prepared in suspended, frozen, centrifuged/concentrated/frozen, or other form. The polypeptide may be locally administered as a semisolid ointment containing, for example, fat, fatty oils, lanolin, vaseline, wax, liquid paraffin, plasters, plastics, glycol, higher alcohols, resins, glycerin, emulsifiers, or suspensions. The ointment may be formed into a sheet, if desired with a hydrophilic polymer, and affixed to the affected area.

The antiobesity agent, antidiabetic agent, or hypolipidemic agent containing the polynucleotide may be administered by selecting an appropriate method and area in accordance with a kind of disease or symptoms. Parenteral administrations are preferable. For example, subcutaneous, intracutaneous, intravascular, or intramuscular administrations, or a surface administration with ointment or the like may be used. Subcutaneous, intracutaneous, intravascular, or intramuscular administrations may be carried out by an injection or catheter.

The medicament of the present invention contains at least one of polynucleotides encoding the polypeptides (a) to (d) in an amount sufficient to cause desired effects for the treatment or prevention of obesity, diabetes, and/or hyperlipemia, i.e., in an amount clinically or pharmaceutically effective therefor. The effective amount in an actual administration may be confirmed, for example, by measuring a degree of recovery from a disease of interest. The actual dose depends on various factors, such as symptoms, age, body weight of each subject to be administered, and preferably may be determined by taking into consideration any noticeable side effects. The dose, toxicity or the like may be easily determined by those skilled in the art by using a known model animal. For example, the usual dosage for an adult is preferably 0.1 µg to 200 mg per day, more preferably 0.1 µg to 20 mg per day.

### [2] Health food of the present invention

The polypeptides (a) to (d) as the active ingredient in the present invention may be administered as a medicament or in various forms, for example, eatable or drinkable products such as health foods (preferably functional foods) or feeds. The term "foods" as used herein includes drinks.

The term "health food" as used herein means food which provides, or is expected to provide, one or more effects on health. The term "functional food" as used herein means processed or designed food such that various biological regulatory functions (for example, functions which regulate physiological systems such as the digestive system, circulatory system, endocrine system, immune system, or nervous system) may be fully expressed.
The health food of the present invention preferably has a function of treating obesity, diabetes, and/or hyperlipemia.
The present invention includes "a composition for food comprising at least one of the polypeptides (a) to (d), characterized by exhibiting an activity of alleviating obesity, diabetes, and/or hyperlipemia" and "a functional food material comprising at least one of the polypeptides (a) to (d), characterized by exhibiting an activity of alleviating obesity, diabetes, and/or hyperlipemia". The composition for food may contain a carrier or other additives which may be used as food.

The health food of the present invention may be prepared in accordance with a convention method for preparing known health foods, except that at least one of the polypeptides (a) to (d) is contained as an active ingredient. The health food of the present invention may be prepared, for example, by adding an effective amount of the active ingredient to a substance to be taken as food. The dose may be determined by taking into consideration various factors, such as a kind or form of food, symptoms, age, or body weight of a subject. For example, the usual dosage for an adult is preferably 1 µg to 2 g per day, more preferably 1 µg to 200 mg per day.

### EXAMPLES

The present invention now will be further illustrated by, but is by no means limited to, the following Examples. In this connection, the following procedures may be performed in accordance with known methods (for example, "Molecular Cloning", Sambrook, J. et al., Cold Spring Harbor Laboratory Press, 1989), unless otherwise specified. Further, when a commercially available reagent or kit is used, procedures may be performed in accordance with a protocol attached thereto.

Knockout animals and transgenic animals may be prepared in accordance with "Manipulating the Mouse Embryo. A Laboratory Manual." 2nd Edition, B. Hogan, R. Beddington, F. Costantini, E. Lacy, Cold Spring Harbor, New York, Cold Spring Harbor Laboratory Press, 1994, unless otherwise specified. Further, chimeric mice may be prepared by using ES cells in accordance with AL Joyner: "Gene Targeting, A Practical Approach", OXFORD UNIVERSITY PRESS, 1993; or Shinich Aizawa, Biomanual series 8, "Gene Targeting, ES saibou wo mochiita henimausu no sakusei", Yodosya, 1994, unless otherwise specified.

### Referential Example 1: Preparation of AGF KO mice

### (1) construction of targeting vector

A targeting vector containing a genomic sequence (5' long arm) at the 5' side of the mouse AGF gene, a pgk promoter, a neomycin resistant gene, a genomic sequence (3' short arm) containing a part of exon 2 and the whole of exon 3 in the mouse AGF gene, and an HSV-tk gene, in this order, was prepared in accordance with the following procedures.

A cDNA corresponding to the full-length of the coding region of mouse AGF protein was prepared by the procedures described in Example 1 of WO03/083114. The cDNA was used as a probe to screen a mouse genomic library (Mouse Genomic, 129 SVJ Library; Stratagene) in accordance with a manual attached thereto. A phage clone containing a sequence of approximately 17.9 kbp (corresponding to 90644 to 108544 in mouse-pub-genome sequence AC073775.2 containing the AGF gene) was isolated and subcloned into plasmid pBluescript (Stratagene). The obtained plasmid clone (pBN2) was digested with restriction enzymes SalI and MfeI to obtain the 5' long arm of approximately 6.2 kbp (containing 90664 to 96900 in mouse-pub-genome sequence AC073775.2). Further, a PCR was carried out using the plasmid pBN2 as a template, together with a primer set [SEQ ID NO: 6 (CTAGACTAGTTGCAAAGGCGTGCGGCGG; artificial sequence) and SEQ ID NO: 7 (CTAGACTAGTGGATCCGCAGGCTTGCTTTGACTTAC; artificial sequence)] to obtain the 3' short arm of approximately 2.0 kbp (containing 105903 to 107914 in mouse-pub-genome sequence AC073775.2). The 5' long arm and the 3' short arm were inserted into the XhoI site and the XbaI site of plasmid pPNT [Cell, 1991, 65(7), 1153-1163], respectively, to construct the targeting vector.

### (2) Preparation of homologous recombinant ES cells

The obtained targeting vector was digested with restriction enzyme NotI, and introduced into ES cell line R1 (Proceedings of the National Academy of Sciences, Vol 90, 8424-8428, 1993) by electroporation. The ES cells were cultured in a medium containing G418 to obtain resistant strains. DNAs were extracted from ES cells, and clones in which only a desired homologous recombination occurred were identified by Southern blotting. More particularly, each DNA was digested with restriction enzyme HindIII, and analyzed by Southern blotting using, as a probe, a DNA consisting of the nucleotide sequence of SEQ ID NO: 8 (GCCCATGGAGGGATTGTGCAGAGGCTCACGGGGCAGGTCACTGGCAGAGTGGAGTGTAT GACCTGCGGCTGGGCCGTCGTGTAGTAGCCGTGTGGTGTGAACAGCAGCAGGAAGTGGAG GCTGGACTGTCATCCAGAGACGGCAGGACGGCTCTGTCAACTTCTTCACCAACTGGCAGC ACTACAAGGTGTGTGCTTGTGGTGGGGGTGTCAGAGACTGCTGGGCAGAGAGGACGCCCC CACCCTCTTCCTCCTACCCTTCCAGGCGGGCTTTGGGCGTCCAGAAGGAGAATACTGGCT GGGCCTGGAACCTGTGCATCAGGTGACAAGCCGTGGGGACCACGAGCTGCTGATACTCCT AGAGGACTGGGGGGGCCGTGCAGCACGCGCCCACTACGACAGCTTCTCCTTGGAGCCTGA GAGTGACCACTACCGTCTGCGGCTTGGCCAGTACCACGGCGATGCCGGAGACTCCCTCTC TTGGCACAATGACAAAACCTTTCAGCACTGTGGATAGGGACAGAGACTCATATTCTG; mouse) containing exon 4 and exon 5 in AGF. As a result, a DNA fragment of 6.5 Kb was detected in homologous recombinant clones, in comparison with that of 4.6 Kb in the wild-type.

### (3) Preparation of AGF KO mice

The obtained ES cell line was microinjected into blastocysts prepared from BDF2 mice which were F2 hybrid mice of C57BL/6 and DBA/2 mice, and the manipulated embryos were transferred to a uterus, to obtain chimeric mice from the pregnant mice. The chimeric mice were mated with C57BL/6 mice to obtain heterozygous mice (hereinafter referred to as AGF heterozygous KO mice) having a mutated allele lacking in the initiation codon of AGF. The AGF heterozygous KO mice were mated with each other to obtain homozygous mice (hereinafter referred to as AGF homozygous KO mice). A PCR using genomic DNA isolated from the tail of each offspring mouse as a template was carried out to confirm the genotype thereof from the size of each DNA fragment obtained by the PCR, as described below. That is, the tail was treated with proteinase K, and a phenol/chloroform extraction was carried out to obtain DNA. The extracted DNA was collected by an isopropanol precipitation followed by an ethanol precipitation, and dissolved in a Tris-EDTA buffer (hereinafter referred to as TE solution). The following primers were designed on the basis of a sequence of the neomycin resistant gene and a genomic sequence to be deleted by targeting:
(neomycin resistant gene)
   Forward primer: SEQ ID NO: 9 (5'-agaggctattcggctatgac-3'; artificial sequence)
   Reverse primer: SEQ ID NO: 10 (5'-caccatgatattcggcaagc-3'; artificial sequence)
(genomic DNA)
   Forward primer: SEQ ID NO: 11 (5'-tggcctctgttatcatgctc-3'; mouse)
   Reverse primer: SEQ ID NO: 12 (5'-ctacctacatccactcctac-3'; mouse)

The genomic DNA obtained from each offspring mouse was used together with the above primers and a DNA polymerase (ExTaq; Takara) to perform PCRs. In the PCRs, a thermal denature at 95°C for 5 minutes was carried out, a cycle composed of reactions at 95°C for 1 minute, at 60°C for 1 minute, and at 72°C for 1 minute was repeated 30 times, and an elongation reaction at 72°C for 7 minutes was carried out. The sizes of fragments amplified by the PCRs were analyzed. When an offspring mouse has the mutated allele, a band of 545 bp is detected in the PCR for detecting the neomycin resistant gene. When an offspring mouse has the wild-type allele, a band of 322 bp is detected in the PCR for detecting the genomic DNA containing mouse AGF exon 1. The genotype of each mouse was determined from the results. As a result, in the AGF homozygous KO mice, the band of the mutated allele was detected, but the band of the wild-type allele was not detected. In the AGF heterozygous KO mice, the band of the mutated allele and that of the wild-type allele were detected. In the wild-type mice (hereinafter referred to as littermate WT mice), the band of the mutated allele was not detected, but the band of the wild-type allele was detected.

Further, the genotype of each mouse was analyzed by Southern blotting described in Referential Example 1(2). As a result, in the AGF homozygous KO mice, a band of 6.5 kbp derived from the mutated allele was detected. In the AGF heterozygous KO mice, a band of 6.5 kbp derived from the mutated allele and that of 4.6 kbp derived from the wild-type allele were detected. In the littermate WT mice, the band of 4.6 kbp derived from the wild-type allele was detected.

Furthermore, each blood sample collected from the AGF KO mice was allowed to stand at 37°C for 30 minutes, and centrifuged to obtain a serum as the supernatant. The serum was diluted to 1/20 with a lysis buffer [0.5 mol/L HEPES (pH7.2), 1% Triton X-100, 10% glycerol, 10 mmol/L Na₄P₂O₇, 0.1 mol/L NaF, 0.1 mmol/L Na₃VO₄, 4 mmol/L EDTA (pH 8), 0.05 mg/mL aprotinin, 1 mmol/L PMSF, 0.1 mmol/L leupeptin, and 0.025 mmol/L Pepstatin A], and further diluted with an equal volume of a 2×SDS sample buffer. Each sample (20 µL per lane) was subjected to 10% acrylamide gel electrophoresis, followed by Western blotting. In the Western blotting, TBS-T [20 mmol/L Tris-HCl (pH 7.5), 150 mmol/L NaCl, and 0.05%(w/v) Tween 20] containing 5% bovine serum albumin (BSA) was used as a blocking agent, an anti-mouse AGF antibody (WO03/083114) was used as the primary antibody, and an anti-rabbit antibody (ALI3404; BIO SourCE) diluted to 1/5000 with TBS-T containing 3% BSA was used as the second antibody. The AGF band was not detected in the AGF homozygous KO mice, to confirm a deficiency of AGF.

### Example 1: Preparation of CAG-AGF Tg mice

In this example, AGF transgenic mice (hereinafter referred to as CAG-AGF Tg mice) in which mouse AGF was systemically overexpressed under the control of a CAG (modified chicken beta-actin promoter with CMV-IE enhancer) promoter [GENE, 108(1991) 193-200] were prepared. A plasmid in which the CAG promoter (1.7 kb), a lox71 sequence, a blasticidin gene (bsr), a poly A signal sequence (0.5 kb), a lox P sequence, a mouse AGF cDNA sequence, and an IRES (internal ribosomal entry site)-β-geo-poly A sequence (4.5 kb) were inserted at the multicloning site of plasmid pBluescriptII KS(+) (Stratagene) in this order was prepared in accordance with the following procedures.

The full-length of mouse AGF cDNA (WO03/083114) was used as a template, together with a primer set [SEQ ID NO: 13 (AGAAGCTTCACCATGGGGACCGCCAGGCTAC; artificial sequence) and SEQ ID NO: 14 (CCGTCGACATTAGATCTTCACAAGCGCACAAGCCGGGTC; artificial sequence)] to carry out a PCR. In the PCR, a reaction at 95°C for 10 minutes was carried out, and a cycle composed of reactions at 94°C for 15 seconds, at 60°C for 30 seconds, and at 72°C for 2 minutes was repeated 45 times. The obtained PCR product was subcloned into a pZErO-2 cloning vector (Invitrogen). The obtained plasmid was digested with restriction enzymes HindIII and SalI, and inserted between the HindIII and SalI sites of plasmid pBluescriptII SK (Stratagene) to construct plasmid pBS-mAGF containing the full-length of mouse AGF gene. To introduce the IRES-β-geo-poly A gene into the plasmid pBS-mAGF, plasmid pU-San (Hum Mol Genet 8:387-396 1999) carrying the IRES-β-geo-poly A gene was digested with restriction enzymes SalI and BglII, and the obtained IRES-β-geo-poly A gene was inserted between the BglII and SalI sites of pBS-mAGF to construct plasmid pBS-mAGF-βgeo containing the mouse AGF cDNA sequence and the IRES-β-geo-poly A sequence.

A plasmid in which bsr and the poly A signal sequence are interposed between the lox71 sequence and the loxP sequence by inserting the loxP sequence into the 3' side of the poly A signal of plasmid pCAGlox71bsr [Nucleic Acids Res. 1997; 25(4): 868-872] carrying the CAG promoter, the lox71 sequence, bsr, and the poly A signal sequence, was constructed as described below. That is, a phosphorylated fragment of 81 bp (SEQ ID NO: 15; GATCCGGAACCCTTAATATAACTTCGTATAATGTATGCTATACGAAGTTATTAGGTCCCT CGACCTGCAGCCCGGGGGATC; artificial sequence) carrying loxP was inserted into the plasmid pCAGlox7lbsr, which had been previously digested with restriction enzyme SmaI and had been treated with BAP (bacterial alkaline phosphatase), to construct plasmid loxP-lox71. To confirm the loxP sequence inserted in the plasmid loxP-lox71, a nucleotide sequence containing the inserted loxP sequence was sequenced using a T3 primer (SEQ ID NO: 16; AATTAACCCTCACTAAAGGG). As a result, it was confirmed that the nucleotide sequence determined by the T3 primer accorded with that of SEQ ID NO: 15, and the loxP and lox71 sequences have the same direction. The plasmid loxP-lox71 was digested with restriction enzyme SpeI, and the obtained fragment containing the CAG promoter, the lox71 sequence, bsr, the poly A signal sequence, and the loxP sequence was inserted at the SpeI site of plasmid pBS-mAGF-βgeo to construct the desired plasmid pBS-loxP-lox71-mAGF-βgeo. The structure of the plasmid pBS-loxP-lox71-mAGF-βgeo is shown in Figure 1. The plasmid pBS-loxP-lox71-mAGF-βgeo was digested with restriction enzyme NotI to obtain a linearized DNA fragment containing the CAG promoter, the lox71 sequence, bsr, the poly A signal sequence, the loxP sequence, the mouse AGF cDNA sequence, and the IRES-β-geo-poly A sequence (pA).

The linearized DNA fragment was introduced into TT2 ES cells [Anal. Biochem., 1993, 214(1): 70-76] by electroporation (0.8 V, 3 µF). The ES cells were cultured in the presence of 4 µg/mL blasticidin, and cells in which the genes were introduced were selected to establish 20 clones. A CAG-Cre vector (Blood, 1326-1333, Vol.100, 2002) as a circular plasmid was introduced into each clone by electroporation (0.8 V, 3 µF).

When Cre recombinase expressed by the CAG promoter excises the gene between lox71 and loxP, AGF and β-geo will be expressed by the CAG promoter activity. To select clones expressing β-geo, the ES cells were cultured in the presence of G418 (200 µg/mL) to select cells having the genetic structure in which the region between lox71 and loxP was deleted. ES cells (15 clones) selected at this stage have the CAG promoter (1.7 kb), the lox71 sequence, the mouse AGF cDNA sequence, and the IRES-β-geo-poly A sequence (4.5 kb), and express AGF constitutively under the control of the CAG promoter. Each original clone before introducing the CAG-Cre vector (i.e., ES cells in which the region between lox71 and loxP was not deleted) was used as a negative control to confirm that AGF was expressed in the selected 15 clone by Western blotting using the anti AGF antibody described in Referential Example 1(3). That is, ES cells were lysed with the lysis buffer, followed by an equal volume of the 2×SDS sample buffer, the obtained samples were subjected to Western blotting, and the expression of AGF was confirmed. From the ES cells expressing AGF, three lines (8-1, 8-2, and 9-1) were selected. The selected cell lines were microinjected into blastocysts, and the manipulated embryos were transferred to a uterus to obtain chimeric mice from the pregnant mice. The chimeric mice were mated with C57BL/6 mice to obtain transgenic mice expressing AGF constitutively under the control of the CAG promoter. To identify the transgenic mice, a PCR using genomic DNA isolated from the tail of each offspring mouse as a template was carried out, as described below. That is, the tail was treated with proteinase K, and a phenol/chloroform extraction was carried out to obtain DNA. The extracted DNA was collected by an isopropanol precipitation followed by an ethanol precipitation, and dissolved in a TE solution.

The following primers were designed on the basis of the mouse AGF cDNA sequence and a LacZ sequence: (AGF)
Forward primer: SEQ ID NO: 17 (5'-cccactacgacagcttctcc-3'; mouse)
Reverse primer: SEQ ID NO: 18 (5'-agccgggtcaacataacagc-3'; mouse)
(LacZ)
Forward primer: SEQ ID NO: 19 (5'-gcgttacccaacttaatcg-3'; artificial sequence)
Reverse primer: SEQ ID NO: 20 (5'-tgtgagcgagtaacaacc-3'; artificial sequence)
In PCRs using these primers, fragments of 325 bp and 320 bp are amplified from the introduced gene in PCRs for detecting the AGF cDNA and for detecting LacZ, respectively, and such fragments are not amplified from the mouse genomic DNA. The above primers and each genomic DNA prepared from the offspring mice were used to perform PCRs using a DNA polymerase (ExTaq; Takara). In the PCRs, a thermal denature at 94°C for 5 minutes was carried out, a cycle composed of reactions at 94°C for 1 minute, at 62°C for 1 minute and 30 seconds, and at 72°C for 1 minute and 30 seconds was repeated 28 times, and an elongation reaction at 72°C for 7 minutes was carried out. The sizes of fragments amplified by the PCRs were analyzed. As a result, the expected bands were detected in both PCRs for detecting the AGF cDNA and LacZ, with respect to the three lines. The result shows that a germ line transmission occurred in the three lines, and that the three lines are transgenic mice expressing AGF constitutively under the control of the CAG promoter.

### Example 2: Expression of AGF gene in CAG-AGF Tg mouse

In this example, the degree of the AGF gene expressed in the CAG-AGF Tg mouse prepared in Example 1 was analyzed. Total RNAs were prepared from the CAG-AGF Tg mouse and the littermate WT mouse [white adipose tissue (WAT), brown adipose tissue (BAT), cerebrum, cerebellum, hypothalamus, heart, liver, kidney, spleen, skeletal muscle, and pancreas] using a trizol reagent (Invitrogen). A commercially available RNA purification reagent (RNeasy; Qiagen) and DNase (Qiagen) were used to perform a DNase treatment and cleanup of the total RNAs. After the DNase treatment, 0.5 µg of the total RNAs was converted to cDNAs using superscript first-strand system for RT-PCR (LIFE TECHNOLOGIES).

Amounts of AGF and 18S ribosomal RNA (18SrRNA) expressed were determined by a quantitative PCR method. The 18SrRNA was used as an internal standard. The quantitative PCR was carried out by measuring an amount of real-time fluorescence using a sequence detection system (ABI PRISM 7900HT Sequence Detection System; Applied Biosystems). The above cDNAs were used as a template, and the following primers and a Taq Man probe designed for each gene were used as primers. Primers [SEQ ID NO: 21 (TCGTGTAGTAGCCGTGTGGTGT; mouse) and SEQ ID NO: 22 (CACCTGATGCACAGGTTCCA; mouse)] and a commercially available PCR reagent (SYBR Green PCR Master Mix; Applied Biosystems) were used to carry out a PCR for measuring an amount of the AGF gene expressed. Primers [SEQ ID NO: 23 (TGGTTGATCCTGCCAGTAG; mouse) and SEQ ID NO: 24 (CGACCAAAGGAACCATAACT; mouse)], a Taq Man probe [SEQ ID NO: 25 (CCGGTACAGTGAAACTGCGAATG; mouse)], and a commercially available PCR reagent (TaqMan Universal PCR Master Mix; Applied Biosystems) were used to carry out a PCR for measuring an amount of the 18SrRNA expressed.

In the PCR, an initial denaturing reaction at 95°C for 10 minutes was carried out, and a cycle composed of reactions at 94°C for 15 seconds and at 60°C for 60 seconds was repeated 45 times. Standard curves for calculating amounts of genes expressed were prepared by using the above cDNAs or mouse genomic DNA as a template. Amounts of genes expressed were calculated as relative values between samples.
As a result, it was revealed that an amount of the AGF gene expressed in tissues of the CAG-AGF Tg mouse was increased in comparison with that in tissues of the WT mouse. In particular, remarkably increased expressions were observed in skeletal muscle, BAT, and heart.

### Example 3: Changes in body weight of genetically modified mice

### (1) Changes in body weight of CAG-AGF Tg mice

Two generations of backcrosses of the CAG-AGF Tg mouse with C57BL/6 were carried out to obtain CAG-AGF Tg mice (F2). The CAG-AGF Tg mice and the littermate WT mice were normally bred with a normal diet (CE-2; CLEA). When the body weights of 6 weeks old female mice were measured and compared, those of the CAG-AGF Tg mice and the WT mice were 15.7 g ± 0.8 g (SD) and 17.8 g ± 0.5 g (SD), respectively. When those of 12 weeks old female mice were measured and compared, those of the CAG-AGF Tg mice and the WT mice were 19.5 g ± 0.7 g (SD) and 23.5 g ± 2.5 g (SD), respectively. It was found from the results that the body weight of the CAG-AGF Tg mouse was lighter than that of the WT mouse.

Changes in body weight when the above mice were bred with a high fat diet were examined. The mice were normally bred with a high fat diet (high fat diet-32; CLEA) for 12 weeks, and changes in body weight were analyzed. As a result, the amounts of body weight increase for 12 weeks were 7.1 g ± 1 g (SD) in CAG-AGF Tg mice and 21.8 g ± 4 g (SD) in WT mice, respectively. The results show that when the CAG-AGF Tg mice were bred with a normal diet, an increase in body weight was suppressed, and that when the CAG-AGF Tg mice were bred with a high fat diet, an increase in body weight was remarkably suppressed. From the results, it was found that AGF exhibits an activity of suppressing an increase in body weight.

### (2) Changes in body weight of AGF KO mice

The AGF homozygous KO mice, the AGF heterozygous KO mice, and the littermate WT mice were normally bred with a normal diet. Each body weight was measured every week until the mice were 24 weeks old. The results are shown in Figure 2. As shown in Figure 2, the body weight of the AGF homozygous KO mice was higher than that of the WT mice from approximately 12 weeks old, and an increase in body weight of the AGF homozygous KO mice continued and the mice became remarkably obese. The AGF heterozygous mice exhibited an intermediate phenotype between those of the AGF homozygous KO mice and the littermate WT mice.

### Example 4: Changes in tissue weight of genetically modified mice

### (1) Changes in tissue weight in CAG-AGF Tg mice

As described above, it was found that an increase in body weight was suppressed in the CAG-AGF Tg mouse. In this example, the weights of various organs were measured to reveal the mechanism. Each tissue [genital fat pads (WAT), brown adipose tissue (BAT), liver, heart, kidney, and spleen] was obtained from the CAG-AGF Tg mice and the littermate WT mice, and the weight of each tissue per body weight was measured. The measurement was carried out using 12 weeks old mice bred with a normal diet and mice bred with a high fat diet for 12 weeks (from 12 weeks old to 24 weeks old). As a result, no changes were observed in BAT, liver, heart, kidney, and spleen between the mice (i.e., CAG-AGF Tg mice and WT mice) bred with a normal diet or a high fat diet. In contrast, the weight per body weight of genital fat pads (white adipose tissue) in the CAG-AGF Tg mice bred with a normal diet or a high fat diet was decreased in comparison with that in the WT mice. The results show that, in the CAG-AGF Tg mouse, an increase in the weight of WAT was suppressed, and thus, an increase in the body weight thereof was suppressed. That is, it was found that AGF does not act on the weights of tissues other than WAT, but suppresses an increase in the weight of adipose tissue accompanied by obesity. The results in WAT are shown in Figure 3 (normal diet) and Figure 4 (high fat diet), respectively.

### (2) Changes in tissue weight in AGF KO mice

As described above, it was found that the body weight was increased in the AGF KO mouse. In this example, the weights of various tissues were measured to reveal the mechanism. Each tissue [genital fat pads (WAT), brown adipose tissue (BAT), liver, heart, kidney, and spleen] was obtained from 20 weeks old female AGF homozygous KO mice, AGF heterozygous mice, and littermate WT mice bred with a normal diet, and the weight of each tissue per body weight was measured. As a result, no changes were observed in BAT, liver, heart, kidney, and spleen among the AGF homozygous KO mice, the AGF heterozygous mice, and the littermate WT mice. In contrast, the weight per body weight or per mouse of genital fat pads in the AGF homozygous mice was increased in comparison with that in the WT mice. The AGF heterozygous mice exhibited tissue weights intermediate between those of the AGF homozygous KO mice and the WT mice. The results show that, in the AGF KO mouse, the weight of WAT such as genital fat pads was increased, and thus, the body weight thereof was increased. That is, it was found that the AGF KO mouse exhibits phenotypes opposite to the CAG-AGF Tg mouse, and that AGF does not act on the weights of tissues other than WAT, but suppresses an increase in the weight of adipose tissue. The results in genital fat pads (WAT) are shown in Figure 5 (weight of WAT) and Figure 6 (weight of WAT/body weight), respectively.

### Example 5: Changes in form of adipocytes in genetically modified mice

### (1) Changes in form of adipocytes in CAG-AGF Tg mice

It is known that a high fat diet increases the weight of WAT and hypertrophy of adipocytes. It is known that the hypertrophy of adipocytes is involved in deteriorating diabetes [IGAKU NO AYUMI, 192, 513-518, 2000; and IGAKU NO AYUMI, 192, 541-545, 2000]. Therefore, the forms of adipocytes in the CAG-AGF Tg mice were analyzed in this example, as described below. Each fat tissue was obtained from CAG-AGF Tg mice and littermate WT mice bred with a high fat diet for 12 weeks from 12 weeks old to 24 weeks old. Each tissue was fixed with a 10% formalin neutral buffer solution (Wako) and embedded in paraffin. Sliced sections were prepared and a hematoxylin and eosin (H&E) stain was carried out. The result is shown in Figure 7. In the littermate WT mouse (NTG), adipocytes became hypertrophied. In the CAG-AGF Tg mouse (TG), the hypertrophy of adipocytes was suppressed and the sizes thereof were maintained as a normal size.

### (2) Changes in form of adipocytes in AGF KO mice

It is known that adipocytes become hypertrophied in model mice for diabetes or obesity, accompanied by an increase in the weight of adipocytes [Diabetologia, 14(3), 141-148, 1978]. It is known that the hypertrophy of adipocytes is involved in deteriorating diabetes, and thus, the forms of adipocytes in the AGF KO mice were analyzed in this example, as described below. Each genital fat pads (WAT) and brown fat tissue (BAT) were obtained from the AGF homozygous mice and the littermate WT mice. Each tissue was fixed with a 10% formalin neutral buffer solution and embedded in paraffin. Sliced sections were prepared and a hematoxylin and eosin (H&E) stain was carried out. The results are shown in Figure 8 (AGF homozygous KO mouse) and Figure 9 (littermate WT mouse), respectively. It was found that adipocytes in WAT of the littermate WT mice had a normal size, and that adipocytes in WAT of the AGF homozygous KO mice became hypertrophied. Further, an accumulation of fat in BAT of the AGF homozygous KO mice was observed in comparison with that of the littermate WT mice.

### Example 6: Changes in triglyceride content in tissues of genetically modified mice

### (1) Changes in triglyceride content in tissues of CAG-AGF Tg mice

It is known that obesity causes not only an increase in fat tissues, but also an increase in triglyceride (TG) content in skeletal muscles or liver (Nippon Rinsho, 1995, vol. 53, Special Issue in 1995, Himansho, p. 354-p358). In this example, TG content in skeletal muscles (gastrocnemial muscles) and liver of the CAG-AGF Tg mice were analyzed, as described below. The CAG-AGF Tg mice (Tg) and the littermate WT mice (WT) were bred with a high fat diet (high fat diet 32; CLEA) for a month or three months. A chloroform-methanol solution was used to extract TG from skeletal muscles and liver of each mouse (Seikagakujikkenkouza 3, Shishitsunokagaku, Tokyo Kagaku Dozin). A concentration of each extracted TG was measured using a kit (Triglyceride E test Wako; Wako) to determine TG content in the tissues. The results are shown in Figure 10 (liver) and Figure 11 (skeletal muscles), respectively. It was found that each TG content in tissues (skeletal muscles or liver) of the CAG-AGF Tg mice was decreased in a comparison with that in the WT mice. It was found that AGF exhibits an activity of decreasing TG content in skeletal muscles or liver. In this connection, it is known that the TG content in skeletal muscles or liver is increased by obesity.

### (2) TG content in tissues of AGF KO mice

TG content in skeletal muscles (gastrocnemial muscles) and liver of the AGF KO mice was analyzed. The method described in Example 6(1) was repeated, except that the AGF KO mice and the littermate WT mice were used, to extract TG therefrom. A concentration of each extracted TG was measured using a kit (Triglyceride E test Wako; Wako) to determine TG content in the tissues. The results are shown in Figure 12. It was found that TG content in skeletal muscles and liver of the AGF KO mice was remarkably increased in comparison of that in the WT mice. It was found that the AGF KO mouse exhibits phenotypes opposite to the CAG-AGF Tg mouse, and that AGF exhibits an activity of decreasing TG content in skeletal muscles or liver.

### Referential Example 2: Changes in blood glucose level and blood insulin concentration in AGF KO mice

A glucose tolerance test for the AGF homozygous KO mice and the littermate WT mice was carried out to analyze a blood glucose level and a concentration of blood insulin, as described below. The mice were made to fast for 16 hours, and 1 g/kg of D-glucose was intraperitoneally administered. Blood was taken from ophthalmic veins before the administration, and at 15, 30, 60, and 120 minutes after the administration. The blood glucose level was measured using GLUTEST ACE (SANWA KAGAKU KENKYUSHO), and the concentration of blood insulin was measured using a RIA2 antibody method (SRL). The results are shown in Figure 13 (blood glucose value) and Figure 14 (insulin in plasma), respectively.

In the WT mice, the blood glucose level increased by the glucose administration began to decrease at 30 minutes after the administration. In the AGF homozygous mice, the blood glucose level was greatly increased by the glucose administration, and the elevated blood glucose value did not begin to decrease at 60 minutes after the administration. It was found from the results that the AGF homozygous KO mice exhibited an abnormality in glucose tolerance. With respect to the concentration in blood insulin, it was increased by the glucose administration in the WT mice, whereas the concentration of blood insulin in the AGF homozygous KO mice was remarkably high before the glucose administration. This shows that the AGF homozygous KO mice suffered from hyperinsulinemia. From the results, it was found that the AGF homozygous KO mouse deficient in the AGF gene suffered from diabetes, and that AGF exhibits an antidiabetic activity.

### Example 7: Oxygen consumption in CAG-AGF Tg mice

The oxygen consumption in the CAG-AGF Tg mice and the littermate WT mice was measured in this example. An apparatus for measuring oxygen consumption (OXYMAX; Columbus Instruments) was used to measure oxygen consumption in fasting mice for 24 hours in accordance with a manual attached to the apparatus. Oxygen consumption in 14 CAG-AGF Tg mice and 14 littermate WT mice was measured to determine and prepare oxygen consumption for a 12-hour light period (7:00-19:00), that for a 12-hour dark period (19:00-7:00), and that for 24 hours. The results are shown in Figure 15. The oxygen consumption (VO₂) in the CAG-AGF Tg mice was higher than that in the WT mice in any time zone. It was found that AGF exhibits an activity for promoting oxygen consumption. Because oxygen consumption correlates with energy consumption, the promotion of oxygen consumption exhibits an antiobesity activity [FEBS Letters, 491(1-2): 154-158, 2001]. It was found that AGF exhibits an activity of promoting oxygen consumption. The result supports the antiobesity activity of AGF.

### Example 8: Changes in genes expressed in tissues of CAG-AGF Tg mice

In this example, changes in a UCP (Uncoupling Protein) gene and a PPAR (Peroxisome Proliferator-Activated Receptor) gene expressed in brown adipose tissue (BAT) and skeletal muscles of the CAG-AGF Tg mice were analyzed. In accordance with the procedures described in Example 2, total RNAs were prepared from BAT and skeletal muscles of the CAG-AGF Tg mice and the littermate WT mice, and were treated with DNase, and cDNAs were synthesized. Amounts of UCP1, UCP3, PPAR-α, PPAR-δ, and β-actin expressed were determined by the quantitative PCR method, described in Example 2. Primers shown in Table 1 were used in the quantitative PCR. Further, as commercially available PCR reagents, SYBR Green PCR Master Mix (Applied Biosystems) was used for β-actin, and TaqMan Universal PCR Master Mix (Applied Biosystems) was used for UCP1, UCP3, PPAR-α, and PPAR-δ.

**Table 1**

| Genes | Forward primer | Reverse primer | TaqMan primer |
|---|---|---|---|
| UCP 1 | SEQ ID NO: 26 | SEQ ID NO: 27 | SEQ ID NO: 28 |
| UCP 3 | SEQ ID NO: 29 | SEQ ID NO: 30 | SEQ ID NO: 31 |
| PPAR-α | SEQ ID NO: 32 | SEQ ID NO: 33 | SEQ ID NO: 34 |
| PPAR-δ | SEQ ID NO: 35 | SEQ ID NO: 36 | SEQ ID NO: 37 |
| β-actin | SEQ ID NO: 38 | SEQ ID NO: 39 | Not used |

As a result, it was found that the expression of UCP1 was induced in BAT of the CAG-AGF Tg mice, and that the expressions of UCP3, PPAR-α, and PPAR-δ were induced in skeletal muscles of the CAG-AGF Tg mice. That is, it was found that AGF induces the expression of UCP1 in BAT and those of UCP3, PPAR-α, and PPAR-δ in skeletal muscles. Accordingly, it was revealed that AGF promotion in expression of UCP promoting heat consumption and in expression of PPAR promoting heat consumption or lipid metabolism is one of the mechanisms of AGF activities for promoting oxygen consumption, suppressing an increase in body weight, and suppressing an increase in the weight of adipose tissues.

### Example 9: Expression and purification of mouse AGF and human AGF

The human AGF and the mouse AGF were expressed and purified in accordance with the procedures described in WO03/083114 (Example 19) as described below. That is, DNA fragments of approximately 1.4 kbp (human) and approximately 1.3 kbp (mouse) were independently inserted into plasmid pcDNA-Signal-FLAG. Each resulting expression plasmid was introduced into HEK293 cells. Each culture supernatant of the cells expressing the human AGF or the mouse AGF was purified by affinity chromatograph using anti FLAG-M2 monoclonal antibody agarose affinity gel (Sigma) to obtain human and mouse recombinant AGF proteins.

### Example 10: Preparation of adenovirus expressing mouse AGF

Plasmid pCR2.1-mNew (prepared in Example 1 of WO03/083114) containing the full-length of mouse AGF cDNA was used as a template, together with a forward primer (AATCTAGACACCATGGGGACCGCCAGGCTACG; SEQ ID NO: 40) and a reverse primer (AAGCGGCCGCCAAGCGCACAAGCCGGGTCAA; SEQ ID NO: 41) and a pfu DNA polymerase, to carry out a PCR. In the PCR, a reaction at 95°C for 10 minutes was carried out, and a cycle composed of reactions at 94°C for 15 seconds, at 60°C for 30 seconds, and at 72°C for 2 minutes was repeated 45 times. The resulting PCR product was subcloned into pCR4Blunt-TOPO (Invitrogen). The obtained plasmid was digested with restriction enzymes XbaI and NotI, and inserted between the XbaI and NotI sites of C-terminal-FLAG-addition-type expression vector pCEP4dE2-FLAG (prepared in accordance with Example 2 of WO02/42448) to obtain pCEPdE2-mAGF-FLAG. Plasmid pCEPdE2-mAGF-FLAG was used as a template, together with a forward primer (GAAGATCTACCATGGGGACCGCCAGGCTACGC; SEQ ID NO: 42) and a reverse primer (CCGCTCGAGTGATGGGACAGTCACAAGCGC; SEQ ID NO: 43) and a PyroBest DNA polymerase, to carry out a PCR. In the PCR, a reaction at 95°C for 2 minutes was carried out, a cycle composed of reactions at 98°C for 20 seconds, at 60°C for 30 seconds, and at 72°C for 1.5 minutes was repeated 20 times, and a reaction at 72°C for 7 minutes was carried out. The resulting PCR product was subcloned into pCR4Blunt-TOPO (Invitrogen). The obtained plasmid was digested with restriction enzymes BglII and XhoI, and the obtained DNA fragment of approximately 1.4 kbp was inserted between the BglII and XhoI sites of a shuttle vector pAdTrack-CMV for preparing adenovirus (Proc. Natl. Acad. Sci. USA 95:2509-2514, 1998) to construct pAdTracK-CMV-mAGF. This plasmid or a control vector pAdTrack-CMV (empty vector) was used to prepare viral particles in accordance with the following procedure.

Escherichia coil BJ5183 (stratagene) was transformed with plasmid pAdEasy-1 (He T.-C. et al., Proc. Natl. Acad. Sci. USA, 95, 2509-2514, 1998) to prepare Escherichia coil BJ5183 containing plasmid pAdEasy-1 [hereinafter referred to as Escherichia coil BJ5183 (pAdEasy-1)]. The previously prepared plasmid pAdTracK-CMV-mAGF or pAdTrack-CMV was digested with restriction enzyme PmeI, and used to transform Escherichia coil BJ5183 (pAdEasy-1). To select a clone in which plasmid pAdEasy-1 and plasmid pAdTracK-CMV-mAGF or pAdTrack-CMV were recombined, plasmids were prepared from obtained transformants, and digested with restriction enzyme PacI to analyze the digestion patterns. Clones in which an extra band of 3 kb or 4.5 kb was observed were selected to obtain desired clones in which the arm regions of the plasmids were recombined.
Concentrations of viral particles were determined in accordance with a method similar to that described in Example 15 of WO02/42448.

### Example 11: Expression of mouse AGF protein using adenovirus

A human fetal kidney cell line 293 (ATCC No.: CRL-1573) was infected with the adenovirus expressing the mouse AGF protein or the control adenovirus pAdTrack-CMV prepared in Example 10 (100 viral particles/cell). More particularly, 3×10⁵ of 293 cells were seeded into wells of a 6-well plate (ASAHI TECHNO GLASS) coated with type I collagen, and cultured overnight in 2 mL of a DMEM medium supplemented with 10% FBS, 100 IU/mL penicillin, and 100 µg/mL streptomycin. Adenoviral particles (6×10⁷ particles) were added, and further cultured overnight. The medium was exchanged with 2 mL of a DMEM medium supplemented with 100 IU/mL penicillin and 100 µg/mL streptomycin. After two days from the infection, each culture supernatant was collected and centrifuged at 3,000 rpm for 5 minutes. Each supernatant was subjected to an SDS-polyacrylamide gel electrophoresis (SDS-PAGE) using a gradient gel (gel concentration = 4-20%; Daiich Pure Chemicals). Western blotting was carried out using an anti-mouse AGF antibody (prepared in Example 5 of WO03/083114) as the primary antibody and an goat anti-rabbit IgG polyclonal antibody labeled with horseradish peroxidase (Biosource) as the second antibody. When the supernatant derived from cells infected with the adenovirus expressing the mouse AGF protein was used as a sample, a band was detected at the position of the deduced molecular weight of the mouse AGF, but no band was detected when the supernatant derived from cells infected with the control adenovirus was used. As a result, it was found that the adenovirus expressing the mouse AGF protein, prepared in Example 10, was used to express the mouse AGF protein by the method described in Example 11.

### Example 12: Preparation of adenovirus expressing human AGF

Plasmid containing the full-length of human AGF cDNA (prepared in Example 18 of WO03/083114; hereinafter referred to as pCR2.1-hNew) was used as a template, together with a forward primer (CCAAGCTTACCATGGGGAAGCCCTGGCTGCGTGCGCTACAG; SEQ ID NO: 44) and a reverse primer (AACTCGAGCAGCTTCAGGGGCCGAATGAGCATGGC; SEQ ID NO: 1) and a PyroBest™ DNA polymerase (Takara), to carry out a PCR. In the PCR, a cycle composed of reactions at 98°C for 20 seconds, at 64°C for 30 seconds, and at 74°C for 3 minutes in the presence of 5% formamide was repeated 35 times. The resulting PCR product was subcloned into pCR4Blunt-TOPO (Invitrogen). The obtained plasmid was digested with restriction enzymes HindIII and XhoI, and the obtained DNA fragment was inserted between the HindIII and XhoI sites of pCEP4 (Invitrogen) to obtain pCEP-hAGF. A DNA fragment of approximately 0.43 kbp obtained by digesting pCEP-hAGF with restriction enzymes KpnI and MluI, and a DNA fragment of approximately 0.98 kbp obtained by digesting an expression plasmid containing the full-length of human AGF cDNA (prepared in Example 19 of WO03/083114; hereinafter referred to as pcDNA-SF-hAGF) with restriction enzymes MluI and XhoI were inserted between the KpnI and XhoI sites of a shuttle vector pAdTrack-CMV for preparing adenovirus (Proc. Natl. Acad. Sci. USA 95:2509-2514, 1998) to construct pAdTracK-CMV-hAGF. This plasmid or a control vector pAdTrack-CMV was used together with a pAdEasy system to prepare viral particles in accordance with methods similar to those described in Examples 14 and 15 of WO02/42448. Concentrations of viral particles were determined in accordance with a method similar to that described in Example 15 of WO02/42448.

### Example 13: Preparation of anti-human AGF antibody

A rabbit was immunized with a purified full-length of human AGF protein (Example 19 of WO03/083114) to obtain an antibody. Immunizations were carried out every two weeks using 500 µg for the first immunization and 250 µg for the second to fourth immunizations. After 2 weeks from the last immunization, antiserum was obtained by exsanguination. The antiserum was subjected to a protein A column (Amersham Bioscience) to purify an IgG antibody (hereinafter referred to as anti-SF-YP-030 IgG).

### Example 14: Expression of human AGF protein using adenovirus

In accordance with the method similar to that described in Example 11, a human fetal kidney cell line 293 was infected with the adenovirus expressing the human AGF protein prepared in Example 12 or the control adenovirus pAdTrack-CMV prepared in Example 10. The method described in Example 11 was repeated, except that the anti-SF-YP-030 IgG prepared in Example 13 was used as the primary antibody, to detect the expression of the human AGF protein in each culture supernatant. As a result, when the supernatant derived from cells infected with the adenovirus expressing the human AGF protein was used as a sample, a band was detected at the position of the deduced molecular weight of the human AGF, but no band was detected when the supernatant derived from cells infected with the control adenovirus was used. It was found that the adenovirus expressing the human AGF protein, prepared in Example 12, was used to express the human AGF protein by the method described in Example 14.

### Example 15: Administration of adenovirus expressing mouse AGF to mice bred with high fat diet

Female C57BL/6 mice (CLEA) were bred with a high fat diet (high fat diet 32; CLEA) from 8 weeks old. The body weight was increased in the mice bred with a high fat diet, and the mice became obese (approximately 54 g) in 38 weeks old, from which adenovirus was administered. The adenovirus expressing the mouse AGF (mAGF-Adeno) or the control adenovirus (Cont-Adeno) prepared in Example 10 was intravenously administered to mice (each group consisting of four mice) at a dose of 5×10⁹ PFU/mouse/week to the tails thereof. After 12 days from the first administration of each adenovirus, the body weight in the mAGF-Adeno administered mice was decreased by 7.3 g on average, and that in the Cont-Adeno administered mice was decreased by 1.4 g on average. It was found that, when the virus containing the AGF gene was administered to the mAGF-Adeno administered mice, the AGF protein was expressed in the living body and the expressed AGF protein caused a decrease in body weight.

Further, these mice were used to carry out a glucose metabolism test. These mice were made to fast for 16 hours, and 0.4 g/kg of D-glucose was intraperitoneally administered. Blood was taken from ophthalmic veins before the administration, and at 15, 30, 60, and 120 minutes after the administration. The blood glucose level was measured using GLUTEST ACE (SANWA KAGAKU KENKYUSHO). The result is shown in Figure 16.
In the mAGF-Adeno administered mice, the blood glucose level was lower than that in the Cont-Adeno administered mice, with respect to all measurement points after the glucose administration, and it was found that glucose tolerance was improved. From the result, it was clarified that glucose tolerance was improved by an action of the AGF protein highly expressed in the mAGF-Adeno administered mice.

### Example 16: blood insulin and lipid concentrations in CAG-AGF Tg mice bred with high fat diet

Six weeks old CAG-AGF Tg mice (prepared in Example 1) and littermate WT mice (female) were bred with a high fat diet for 12 weeks. Blood was taken from ophthalmic veins.
A concentration of insulin in plasma was measured using Insulin Immunoassay (Eiken Chemical), a concentration of cholesterol in serum was measured using L type Wako Cholesterol (Wako), and a concentration of free fatty acids in serum was measured using NEFA C-Test Wako (Wako), in accordance with protocols attached thereto. These results are shown in Figure 17 (plasma insulin concentration, ng/mL), Figure 18 (serum cholesterol concentration, mg/dL), and Figure 19 (serum free fatty acids concentration, µEq/L).
As a result, it was found that the insulin concentration, the cholesterol concentration, and the free fatty acids concentration in the CAG-AGF Tg mice (TG) were lower than those in the littermate WT mice (NTG). From the result, it was clarified that insulin sensitivity was increased and lipid metabolism was improved by an action of the AGF protein highly expressed in the CAG-AGF Tg mice.

### Example 17: Glucose metabolism test in CAG-AGF Tg mice

A glucose tolerance test was carried out using the CAG-AGF Tg mice (Example 1) to evaluate the effect of the AGF on alleviating glucose tolerance. Four-month-old female CAG-AGF Tg mice and littermate WT mice were made to fast for 16 hours, and 1 g/kg of D-glucose was intraperitoneally administered. Blood was taken from ophthalmic veins before the administration, and at 15, 30, 60, and 120 minutes after the administration. The blood glucose level was measured using GLUTEST ACE (SANWA KAGAKU KENKYUSHO). The result is shown in Figure 20.
In the CAG-AGF Tg mice, the blood glucose level was lower than that in the littermate WT mice, with respect to all measurement points after the glucose administration, and it was found that glucose tolerance was improved. From the result, it was clarified that glucose tolerance was improved by an action of the AGF protein highly expressed in the CAG-AGF Tg mice.

Next, 0.75 units/kg of insulin was intraperitoneally administered to four-month-old female CAG-AGF Tg mice and littermate WT mice. Blood was taken from ophthalmic veins immediately after the insulin administration, and at 20, 40, and 60 minutes after the insulin administration. The blood glucose level was measured using GLUTEST ACE (SANWA KAGAKU KENKYUSHO). The ratio of the blood glucose level after the insulin administration to that before the insulin administration is shown in Figure 21.
In the CAG-AGF Tg mice, the blood glucose level was remarkably decreased in comparison with that in the littermate WT mice, with respect to all measurement points after the insulin administration, and it was found that insulin sensitivity was increased. It was clarified from the result that insulin sensitivity was increased by an action of the AGF protein highly expressed in the CAG-AGF Tg mice.

### Example 18: Changes in body weight and fat weight in K14-AGF Tg mice

A transgenic mouse overexpressing AGF under the control of a K14 promoter was prepared (Example 8 of WO03/083114).
In this transgenic mouse, the AGF protein is overexpressed in epidermal cells, and angiogenesis and tissue generation are caused by an action of the expressed AGF protein. It was confirmed that the AGF overexpressed in epidermal cells exhibited an antiobesity activity and an antidiabetic activity, as shown in Examples 3, 4, and 17 using mice systemically overexpressing the AGF.

The body weights of 8-month-old female K14-AGF Tg mice and littermate WT mice (each group consisting of 5 mice) were measured. The body weight of the K14-AGF Tg mice was approximately 21 g on average, and was decreased in comparison with that (approximately 31 g on average) of the littermate WT mice. Computed tomographic images of the area from the diaphragm to the bottom of the abdominal cavity of the K14-AGF Tg mice and littermate WT mice were obtained at intervals of 2 mm using an X-ray CT for laboratory animals (Aloka), and the weights of visceral fat tissue and subcutaneous fat tissue were determined. The result is shown in Figure 22 (weight of visceral fat tissue) and Figure 23 (weight of subcutaneous fat tissue).
With respect to both visceral fat and subcutaneous fat, the weights of fat tissues of the K14-AGF Tg mice were remarkably decreased in comparison with those of the littermate WT mice. It was clarified from the result that the expressed AGF exhibited an antiobesity activity and an activity of decreasing fat tissue, as similarly shown in the above-mentioned case using the CAG-AGF Tg mice.

### Example 19: Insulin sensitivity test in K14-AGF Tg mice

The insulin sensitivity of the K14-AGF Tg mice was examined. To 8-month-old female K14-AGF Tg mice and littermate WT mice, 0.75 units/kg of insulin was intraperitoneally administered. Blood was taken from ophthalmic veins immediately after the insulin administration, and at 20, 40, 60, 80, and 100 minutes after the insulin administration. The blood glucose level was measured using GLUTEST ACE (SANWA KAGAKU KENKYUSHO). The ratio of the blood glucose level after the insulin administration to that before the insulin administration is shown in Figure 24.
In the K14-AGF Tg mice, the blood glucose level was remarkably decreased in comparison with that in the littermate WT mice, with respect to all measurement points after the insulin administration, and it was found that insulin sensitivity was increased. It was clarified from the result that insulin sensitivity was increased by an action of the AGF protein highly expressed in the CAG-AGF Tg mice.

### Example 20: Administration of adenovirus expressing human AGF to mice bred with high fat diet

It was shown in Example 15 that the mouse AGF exhibited an activity of suppressing a decrease in body weight and an activity of improving glucose tolerance. In this example, it was confirmed that the human AGF exhibited the same activities by carrying out the following tests.
The adenovirus expressing the human AGF (hAGF-Adeno) prepared in Example 12 and the control adenovirus (Cont-Adeno) were administered to mice (each group consisting of three mice) in accordance with a method similar to that described in Example 15. After 13 days from the first administration, the body weight in the hAGF-Adeno administered mice was decreased by 4.6 g on average, and that in the Cont-Adeno administered mice was increased by 0.1 g on average.
Next, the glucose metabolism test described in Example 15 was repeated, except that 0.5 g/kg of D-glucose was intraperitoneally administered and that blood was taken at 30, 60, 90, and 120 minutes after the administration. The result is shown in Figure 25. In the hAGF-Adeno administered mice, the blood glucose level was lower than that in the Cont-Adeno administered mice, with respect to all measurement points after the glucose administration, and it was found that glucose tolerance was improved.
It was clarified from these results that an action of the hAGF protein highly expressed in the hAGF-Adeno administered mice caused a decrease in body weight, and improved glucose tolerance.

### Example 21: AGF protein content in blood of CAG-AGF Tg mice

The CAG-AGF Tg mice (Example 1) were used to examine the amount of the AGF protein expressed in blood. Blood was taken from ophthalmic veins of four-month-old female CAG-AGF Tg mice and littermate WT mice, to prepare sera in accordance with a conventional method. The obtained sera were subjected to Western blotting, in accordance with a method similar to that described in Referential Example 1(3), to detect the AGF protein. Each band detected by the Western blotting was read using a scanner, and the intensities of the bands (i.e., AGF protein) were converted into numbers using an image analysis software (NIH image 1.62f). As a result, the concentration of the serum AGF protein was increased in the CAG-AGF Tg mice by a factor of 2.4, in comparison with that in the littermate WT mice.

### Example 22: AGF protein content in blood of mice to which adenovirus expressing mouse AGF was administered

In Example 15, the adenovirus expressing the mouse AGF was administered to mice. In this Example, the content of the AGF protein in blood was examined. Blood was taken from ophthalmic veins of mice to which the adenovirus expressing the mouse AGF or control adenovirus was administered, before the administration (day 0) and after 1 to 7 days from the administration, to prepare sera in accordance with a conventional method. The obtained sera were subjected to Western blotting, in accordance with a method similar to that described in Referential Example 1(3), to detect the AGF protein. As a result, although the concentration of the AGF protein did not change in the control adenovirus administered mice, the concentration of the AGF protein was increased with time and peaked after 4 days from the administration in the mice to which the adenovirus expressing the mouse AGF was administered. In accordance with a method similar to that described in Example 21, the intensities of the bands (i.e., AGF protein) from the mice to which the adenovirus expressing the mouse AGF at day 0 and day 4 were converted into numbers. As a result, the concentration of the AGF protein was increased by a factor of 2.5 in the mice to which the adenovirus expressing the mouse AGF.

### Example 23: AGF protein content in blood in K14-AGF Tg mice

The K14-AGF Tg mice used in Examples 18 and 19 were used to examine the amount of the AGF protein expressed in blood. Blood was taken from ophthalmic veins of eight-month-old female K14-AGF Tg mice and littermate WT mice, to prepare sera in accordance with a conventional method. The obtained sera were subjected to Western blotting, in accordance with a method similar to that described in Referential Example 1(3), to detect the AGF protein, and each band was converted into numbers. As a result, the concentration of the serum AGF protein was increased in the CAG-AGF Tg mice by a factor of 1.8, in comparison with that in the littermate WT mice.

### Example 24: Preparation of anti-human AGF antibody

A peptide (CSRMLDPAPEPQRDQTQR; SEQ ID NO: 46; hereinafter referred to as peptide A) in which a Cys residue was added to the N-terminus of a peptide having a partial sequence (SRMLDPAPEPQRDQTQR; SEQ ID NO: 45) of the human AGF was synthesized and purified. An antigen was prepared by coupling peptide A with a carrier protein, bovine thyroglobulin (SIGMA), and a rabbit was immunized with 100 µg of this peptide eight times at intervals of 2 weeks. After 1 week from the last immunization, antiserum was obtained by exsanguination. Peptide A was used to prepare an affinity column. The serum was subjected to the affinity column to purify an IgG antibody (hereinafter referred to as anti-YP-030-A IgG) which binds to peptide A. The concentration of the antibody was determined by measuring an absorbance at 280 nm (molar absorption coefficient = 1.38).

### Example 25: HRP labeling of anti-SF-YP-030 IgG

To the anti-SF-YP-030 IgG (prepared in Example 13) dialyzed in a 0.1 mol/L citrate buffer, pepsin (SIGMA) was added while stirring, and the antibody was partially digested at 37°C for 1 hour. The reaction solution was subjected to a gel filtration using a Superdex 200 16/60HR column (Amersham Bioscience) equilibrated with a 0.1 mol/L phosphate buffer (pH 6.0) to collect F(ab')₂. The obtained F(ab')₂ was reduced with 2-mercapto-ethylamine hydrochloride (Nacalai Tesque) at 37°C for 90 minutes, and subjected to a gel filtration using an ULTROGEL ACA54 column (BIOSEPRA) to collect Fab'. A crosslinking agent EMCS [N-(6-Maleimidocaproyloxy)succinimide; DOJINDO] was reacted with horseradish peroxidase (HRP; TOYOBO) at 37°C for 60 minutes. The reaction solution was subjected to a gel filtration using a P6-DG column (BIORAD) equilibrated with a 0.1 mol/L phosphate buffer (pH 6.0) to collect a fraction containing a conjugate of EMCS and HRP (hereinafter referred to as EMCS-HRP). The Fab' was reacted with EMCS-HRP at 4°C for 16 hours. The reaction solution was subjected to a gel filtration using an Superdex 200 16/60HR column to collect a fraction containing a conjugate of Fab' and HRP and obtain an HRP-labeled anti-human AGF antibody Fab' fragment (HRP-anti-SF-YP-030 Fab'). The concentration of the antibody was determined by measuring an absorbance at 280 nm (molar absorption coefficient = 1.38).

### Example 26: Construction of ELISA system for detecting human AGF

To each well of a microplate for ELISA (NUNC), 100 µL/well of 20 µg/mL anti-YP-030-A IgG prepared in Example 24 was added, and allowed to stand at 4°C overnight. The solution in each well was removed by suction. After each well was washed twice with PBS (phosphate-buffered saline), 300 µL/well of a blocking solution (1% BSA, 0.05% NaN₃, and PBS) was added to each well, and allowed to stand at 4°C overnight to carry out a blocking treatment. After each well was washed twice with a washing solution (10 mmol/L phosphate buffer supplemented with 0.05% Tween20), 100 µL/well of double-diluted series of a standard human AGF purified protein was added to each well to carry out a reaction at 37°C for 1 hour. In this connection, the double-diluted series was prepared by sequentially double-diluting the standard human AGF purified protein with a diluent (PBS supplemented with 1% BSA and 0.05% Tween20) from 100 ng/mL to 0.39 ng/mL. After each well was washed seven times with the washing solution, 100 µL/well of an HRP-labeled antibody solution was added to each well to carry out a reaction at 37°C for 0.5 hour. In this connection, the HRP-labeled antibody solution was prepared by diluting the HRP-labeled antibody solution (HRP-anti-SF-YP-030 Fab') prepared in Example 25 with the diluent to a concentration of 1 µg/mL. After each well was washed nine times with the washing solution, 100 µL/well of a TMBZ substrate solution (IBL) was added to each well to carry out a reaction at room temperature for 30 minutes. The developing reaction was stopped by adding 100 µL/well of a stopping solution (1N H₂SO₄) to each well. An absorbance at A450 nm was measured using an absorptiometer (Spectramax). The measurement values were analyzed using a program attached thereto, and a calibration curve could be prepared on the basis of a concentration range having linearity. As above, an ELISA system for detecting the human AGF could be constructed.

### Example 27: Human AGF protein content in blood of mice to which adenovirus expressing human AGF was administered

The mice to which the adenovirus expressing the human AGF was administered were used to examine the amount of the AGF protein in blood. The adenovirus expressing the human AGF (hAGF-Adeno) prepared in Example 12 and the control adenovirus (Cont-Adeno) were administered to mice bred with a high fat diet (each group consisting of six mice) in accordance with a method similar to that described in Example 20. As a result, a decrease in body weight and a improvement of glucose tolerance were observed in the hAGF-Adeno administered mice, as similar to the case shown in Example 20. After 4 days from the adenovirus administration, blood was taken from ophthalmic veins to prepare sera in accordance with a conventional method. The concentration of the serum human AGF protein was measured using the ELISA system constructed in Example 26. Whereas the human AGF protein was not detected in the sera prepared from the Cont-Adeno administered mice, the human AGF protein was detected in the sera prepared from the hAGF-Adeno administered mice at a concentration range of 0.7 to 1.2 µg/mL. The result indicates that desired activities of decreasing body weight and improving glucose tolerance can be obtained when the concentration of the AGF protein in blood is 0.7 to 1.2 µg/mL.

### INDUSTRIAL APPLICABILITY

The medicament of the present invention may be applied to the use in the treatment and/or prevention of obesity, diabetes, and/or hyperlipemia.
Although the present invention has been described with reference to specific embodiments, various changes and modifications obvious to those skilled in the art are possible without departing from the scope of the appended claims.

### FREE TEXT IN SEQUENCE LISTING

Features of "Artificial Sequence" are described in the numeric identifier <223> in the Sequence Listing. Each of the nucleotide sequences of SEQ ID NOS: 1, 6, 7, 9, 10, 13, 14, 16, 19, 20, and 40 to 44 is an artificially synthesized primer sequence, and the nucleotide sequence of SEQ ID NO: 15 is a sequence containing loxP. The amino acid sequence of SEQ ID NO: 46 is a sequence of Peptide A.

## Claims

1. An antiobesity agent, an antidiabetic agent, and/or a hypolipidemic agent comprising as an active ingredient a polypeptide selected from the group consisting of the following (a) to (d) or a polynucleotide encoding the polypeptide:
(a) a polypeptide exhibiting an activity of suppressing an increase in body weight, and comprising the amino acid sequence consisting of amino acids 1-450 of SEQ ID NO: 3 or the amino acid sequence consisting of amino acids 1-433 of SEQ ID NO: 5,
(b) a polypeptide exhibiting an activity of suppressing an increase in body weight, and comprising an amino acid sequence in which 1 to 10 amino acids are substituted, deleted and/or inserted in the amino acid sequence consisting of amino acids 1-450 of SEQ ID NO: 3 or the amino acid sequence consisting of amino acids 1-433 of SEQ ID NO: 5,
(c) a polypeptide exhibiting an activity of suppressing an increase in body weight, and encoded by a DNA which hybridizes under stringent conditions to a DNA encoding the amino acid sequence consisting of amino acids 1-450 of SEQ ID NO: 3 or amino acids 1-433 of SEQ ID NO: 5, and
(d) a polypeptide exhibiting an activity of suppressing an increase in body weight, and comprising an amino acid sequence having a 95% or more identity with that consisting of amino acids 1-450 of SEQ ID NO: 3 or amino acids 1-433 of SEQ ID NO: 5.

2. A functional food or a health food for alleviating obesity, diabetes, and/or hyperlipemia, comprising the polypeptide of claim 1 as an active ingredient.

3. A method for treating or preventing obesity, diabetes, and/or hyperlipemia, comprising administering to a subject in need thereof the polypeptide of claim 1 or a polynucleotide encoding the polypeptide in an amount effective therefor.

4. Use of the polypeptide of claim 1 or a polynucleotide encoding the polypeptide in the manufacture of a medicament for treating obesity, diabetes, and/or hyperlipemia.
